# FASCICULE DE BREVET EUROPEEN

(11) **EP 2 560 963 B1**
(45) Date de publication et mention de la délivrance du brevet: **05.10.2016**
(21) Numéro de dépôt: 11714629.0
(22) Date de dépôt: 15.04.2011
(51) Int. Cl.: C07D 401/04, A61K 31/53, A61P 3/06

(54) **DERIVES DE PYRAZINES EN TANT QU'INHIBITEURS DE L'ENZYME SCD-1 POUR LE TRAITEMENT DES MALADIES DE CANCER OU DU DIABETE**
PYRAZIN DERIVATE ALS HEMMSTOFFE DES ENZYMS SCD-1 ZUR BEHANDLUNG VON KREBSERKRANKUNGEN UND VON DIABETES.
PYRAZINE DERIVATIVES AS INHIBITORS OF THE ENZYME SCD-1 FOR THE TREATMENT OF CANCER DISEASES AND DIABETES

(30) Priorité: 19.04.2010 FR 1052943
(43) Date de publication de la demande: 27.02.2013
(73) Titulaire: Pierre Fabre Médicament, 92100 Boulogne-Billancourt (FR)
(72) Inventeur: LEROY, Isabelle, F-31270 Frouzins (FR); DUPONT-PASSELAIGUE, Elisabeth, F-31770 Colomiers (FR); MIALHE, Samuel, F-81100 Castres (FR); JUNQUERO, Didier, F-81100 Castres (FR); VALEILLE, Karine, F-91120 Palaiseau (FR)
(74) Mandataire: Regimbeau
(86) Numéro de dépôt international: PCT/EP2011/056066
(87) Numéro de publication internationale: WO 2011/131593

(56) Documents cités:
- WO-A1-2008/046226
- WO-A1-2008/089580
- WO-A1-2009/019566
- WO-A1-2010/006962
- DATABASE CHEMCATS [Online] 8 février 2010 (2010-02-08), XP002599128, extrait de STN Database accession no. RN 1185692-74-3

## Description

La présente invention a pour objet des dérivés d'hétérocycles azotés inhibant l'activité de l'enzyme SCD-1 et leur application en thérapeutique humaine.

La Stéaroyl-CoA Désaturase-1 (SCD-1), aussi appelée Δ9-désaturase, est une enzyme limitante de la synthèse d'acides gras mono-insaturés sous le contrôle du facteur de transcription SREBP_{1c} (Miyazaki, M., Kim, Y.C., Ntambi, J.M. A lipogenic diet in mice with a disruption of the stearoyl-CoA desaturase-1 gene reveals a stringent requirement of endogenous monounsaturated fatty acids for triglyceride synthesis. J Lipid Res 42, 1018-1024 (2001)). Ces acides gras mono-insaturés sont impliqués dans la biosynthèse des phospholipides, triglycérides, esters de cholestérol et esters cireux (Dobrzyn, A. ; Ntambi, J. M. Stearoyl-CoA desaturase as a new drug target for obesity treatment. Obesity reviews, 6, 169-174 (2005)).

L'invalidation du gène SCD-1 chez la souris la rend résistante à l'obésité génétique ou induite par la diète; les effets périphériques de la leptine sur l'augmentation de la dépense énergétique, la perte de poids et la sensibilité à l'insuline sont inversement corrélés à l'expression du gène SCD-1 et à l'activité enzymatique (Cohen, P., Miyazaki, M., Socci, N.D. et al. Role for stearoyl-CoA desaturase-1 in leptin-mediated weight loss. Science 297, 240-243 (2002), Ntambi, J.M., Miyazaki, M., Stoehr, J.P. et al. Loss of stearoyl-CoA desaturase-1 function protects mice against adiposity. Proc Natl Acad Sci 99, 11482-11486 (2002), Biddinger, S.B., Miyazaki, M., Boucher, J. et al. Leptin suppresses stearoyl-CoA desaturase-1 by mechanisms independent of insulin and sterol regulatory element-binding protein-1c. Diabetes 55, 2032-2041 (2006)).

L'implication de SCD-1 dans la pathogenèse de l'obésité, est renforcée par la corrélation entre la concentration plasmatique en acide palmitoléique et l'adiposité abdominale chez les enfants (Okada, T., Furuhashi, N., Kuromori, Y. et al. Plasma palmitoleic acid content and obesity in children. Am J Clin Nutr 82, 747-750 (2005)), ainsi que l'association de la surexpression de SCD-1 dans le muscle squelettique chez les adultes obèses avec une mauvaise répartition des acides gras qui entraîne l'inhibition de la β-oxydation hépatique (Hulver, M.W., Berggren, J.R., Carper, M.J. et al. Elevated stearoyl-CoA desaturase-1 expression in skeletal muscle contributes to abnormal fatty acid partitioning in obese humans. Cell Metab 2, 251-261 (2005)). Le ratio plasmatique 18:1/18:0, aussi appelé "index de désaturation", apparaît comme le biomarqueur de l'activité de SCD-1 chez l'homme et corrèle avec le taux de triglycérides plasmatiques et de manière inversement proportionnelle avec le taux de HDL (Attie, A.D., Krauss, R.M., Gray-Keller, M.P. et al. Relationship between stearoyl-CoA desaturase activity and plasma triglycerides in human and mouse hypertriglyceridemia. J Lipid Res 43, 1899-1907 (2002)).

Par ailleurs, SCD1 et la voie de la lipogénèse sont exprimées dans les glandes sébacées humaines (Harrison, W.J., Bull, J.J., Seltmann, H. et al. Expression of lipogenic factors Galectin-12, Resistin, SREBP-1, and SCD in human sebaceous glands and cultures sebocytes. J Invest Dermatol 127, 1309-1317 (2007)) ainsi que chez la souris. Les souris dont le gène SCD-1 est muté (Asebia) ou invalidé (« knockout ») présentent une atrophie des glandes sébacées, une réduction de la production de sébum et des modifications qualitatives de sébum sécrété (Cohen, P., Miyazaki, M., Socci, N.D. et al. Role for stearoyl-CoA desaturase-1 in leptin-mediated weight loss. Science 297, 240-243 (2002), Zheng, Y., Eilertsen, K.J., Ge, L. et al. SCD1 is expressed in sebaceous glands and is disrupted in the asebia mouse. Nat Genet 23, 268-270 (1999), Sundberg, J.P., Boggess, D., Sundberg, B.A. et al. Asebia-2J (Scd1 ab2j): a new allele and model for scaring alopecia. Am J Pathol 156, 2067-2075 (2000)).

L'intérêt de la cible SCD-1 en dermatologie est supporté par le fait que l'hyper-séborrhée est un facteur essentiel impliqué dans la physiopathologie de l'acné. Ce rationnel est renforcé par la revendication récente d'inhibiteurs de SCD-1 en dermatologie vis-à-vis d'indications telles que l'acné, la rosacée ou l'hyperséborrhée (US2008280916) ainsi que l'inhibition de la production de sébum (WO2009019566).

L'implication de SCD1 en oncologie est suggérée par la prédisposition chez les rongeurs (souris, rats) de fonds génétiques particuliers qui présentent une forte expression de SCD1 (4 à 10 fois augmentée) associée à une hépatocarcinogénèse d'une part (Falvella, F.S., Pascale, R.M., Gariboldi, M. et al. Stearoyl-CoA desaturase (Scd1) gene overexpression is associated with genetic predisposition to hepatocarcinogenesis in mice and rats. Carcinogenesis 23, 1933-1936 (2002)), et par le role d'acides gras mono-insaturés produits par SCD1 dans la prolifération et la capacité d'invasion de lignées cancéreuses d'autre part (Scaglia, N. and Igal, R.A. Stearoyl-CoA desaturase is involved in the control of proliferation, anchorage-independent growth, and survival in human transformed cells. J Biol Chem 280, 25339-25349 (2005)). A l'inverse, l'invalidation de l'expression de SCD1 (« knock-down ») par une stratégie anti-sens réduit la prolifération, stimule l'apoptose et prévient l'invasion de cellules d'adénocarcinome de poumon *in vitro* et *in vivo,* ce qui démontre l'intérêt de l'inhibition de SCD1 comme cible anticancéreuse (Scaglia, N. and Igal, R.A. Inhibition of stearoyl-CoA desaturase 1 expression in human lung adenocarcinoma cells impairs tumorigenesis. Int J Oncol 33, 839-850 (2008)). De plus, l'inactivation de la lipogénèse *de novo* par des inhibiteurs chimiques de SCD1 (intervention pharmacologique) confirme la pertinence de cette stratégie (Scaglia, N.,Chisholm, J.W., Igal, R.A. Inhibition of stearoylCoA desaturase-1 inactivates acetyl-CoA carboxylase and impairs proliferation in cancer cells : role of AMPK. PLoS one 4, e6812-1-13 (2009) ; Fritz, V., Benfodda, Z., Rodier, G. et al. Abrogation of de novo lipogenesis by stearoyl-CoA desaturase 1 inhibition interferes with oncogenic signaling and blocks prostate cancer progression in mice. Mol Cancer Ther 9, 1740-1754 (2010)).

En conséquence, l'inhibition de SCD-1 apparaît comme une cible thérapeutique de choix dans le traitement de l'obésité, du diabète de type 2 et des désordres lipidiques liés au syndrome métabolique ainsi que pour le traitement du cancer et également en dermatologie dans les désordres lipidiques de la peau.

Nous avons précédement décrit des composés inhibant l'activité de l'enzyme SCD-1 (FR2933979 / WO2010006962). Toutefois, la modification de la terminaison a permis de mettre en évidence, de manière tout à fait surprenante, des composés plus puissants. La présente invention concerne ces nouveaux dérivés d'hétérocycles azotés inhibant l'activité de l'enzyme SCD-1, leur préparation et leur application en thérapeutique humaine.

Ces composés correspondent à la formule générale I : 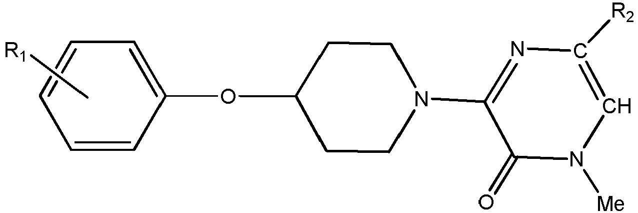 dans laquelle :
- R₁ représente un ou plusieurs substituant(s) choisi(s) parmi trifluorométhtyle, F, Cl, Br, méthyle et nitro,
- R₂ représente Cl ou H
ainsi que leurs sels pharmaceutiquement acceptables.

La présente invention concerne les composés de formule générale I caractérisés en ce qu'ils sont choisis parmi :
1. 5-Chloro-3-[4-(2-chloro-phénoxy)-pipéridin-1-yl]-1-méthyl-1H-pyrazin-2-one
2. 5-Chloro-1-méthyl-3-[4-(2-trifluorométhyl-phénoxy)-pipéridin-1-yl]-1H-pyrazin-2-one
3. 5-Chloro-3-[4-(2-chloro-5-trifluorométhyl-phénoxy)-pipéridin-1-yl]-1-méthyl-1H-pyrazin-2-one
4. 5-Chloro-1-méthyl-3-(4-o-tolyloxy-pipéridin-1-yl)-1H-pyrazin-2-one
5. 1-Méthyl-3-(4-o-tolyloxy-pipéridin-1-yl)-1H-pyrazin-2-one
6. 1-Méthyl-3-[4-(2-trifluorométhyl-phénoxy)-pipéridin-1-yl]-1H-pyrazin-2-one
7. 5-Chloro-3-[4-(2-chloro-5-fluoro-phénoxy)-pipéridin-1-yl]-1-méthyl-1H-pyrazin-2-one.

L'objet de l'invention concerne également les sels pharmaceutiquement acceptables des composés de formule générale I.

La présente invention s'étend également aux procédés de préparation chimique des composés de formule générale I.

La présente invention concerne également les composés de formule générale I et leurs sels pharmaceutiquement acceptables pour leur utilisation en tant qu'inhibiteur de l'enzyme SCD-1.

La présente invention concerne également les composés de formule générale I et leurs sels pharmaceutiquement acceptables pour leur utilisation en tant que médicament.

La présente invention concerne également les composés de formule générale I et leurs sels acceptables en cosmétique pour leur utilisation en tant que principe actif cosmétique.

L'invention concerne aussi les composés de formule générale I et leurs sels pharmaceutiquement acceptables pour leur utilisation en tant que médicament destiné au traitement et/ou la prévention des maladies nécessitant des inhibiteurs de l'activité de l'enzyme SCD-1.

L'invention concerne aussi les composés de formule générale I et leurs sels pharmaceutiquement acceptables pour leur utilisation en tant que médicament destiné au traitement et/ou la prévention des maladies telles que l'obésité, le diabète de type 2, les dyslipidémies diabétiques, l'hypertriglycéridémie, l'hypercholestérolémie, le syndrome métabolique, l'athérosclérose et ses complications, la stéatose hépatique ou les risques cardiovasculaires.

L'invention concerne aussi les composés de formule générale I et leurs sels pharmaceutiquement acceptables, pour leur utilisation en tant que médicament destiné au traitement et/ou à la prévention des :
- états pathologiques liés à des désordres lipidiques de la peau et aux complications inflammatoires et microbiennes;
- dérèglements de la production et/ou de la sécrétion de sébum associés à une hyperandrogénie (quelle qu'en soit l'origine = iatrogène, surrénaliennes, ou ovariennes)

Les maladies dermatologiques liées à un désordre lipidique de la peau sont par exemple l'acné, le psoriasis, l'hirsutisme, la rosacée, la dermite séborrhéique, l'hyperséborrhée, ou l'eczéma.

L'invention concerne aussi les composés de formule générale (I) ainsi que les sels d'addition avec les bases et les acides pharmaceutiquement acceptables, et les différents isomères, ainsi que leurs mélanges en toutes proportions pour leur utilisation en tant que médicament destiné au traitement et/ou la prévention du cancer.

En particulier, par cancer on entend des tumeurs liquides et/ou des tumeurs solides, tels que les mélanomes, les cancers colorectaux, les cancers du poumon, de la prostate, de la vessie, du sein, de l'utérus, de l'oesophage, de l'estomac, du pancréas, du foie, les cancers ovariens, les leucémies en particulier les lymphomes et les myélomes, les cancers de la sphère ORL et les cancers du cerveau.

L'invention s'étend également aux compositions caractérisées en ce qu'elles contiennent à titre de principe actif un composé de formule générale I ou l'un de ses sels pharmaceutiquement acceptable.

L'invention concerne également une composition pharmaceutique caractérisée en ce qu'elle contient un composé de formule générale I ou l'un de ses sels pharmaceutiquement acceptable en association avec tout excipient approprié.

L'invention concerne également une composition cosmétique caractérisée en ce qu'elle contient un composé de formule générale I ou l'un de ses sels acceptable en cosmétique en association avec tout excipient approprié.

La composition pharmaceutique selon l'invention peut être administrée en association avec un anti-diabétique tel que les biguanides (par exemple metformine), les diverses formes d'insuline, les sulfonylurées (par exemple carbutamide, glibornuride, glipizide, gliclazide, glibenclamide, glimepiride), les meglitinides, les modulateurs de PPAR (par exemple pioglitazone), les inhibiteurs de l'alpha-glucosidase (par exemple acarbose, miglitol, voglibose), les inhibiteurs de DPP-4 (par exemple sitagliptin, vildagliptin), les analogues d'amyline (par exemple pramlintide), les analogues de glucagon-like peptide-1 (par exemple exenatide, liraglutide), les inhibiteurs de SGLT2 ou les inhibiteurs de 11β-HSD1.

La composition pharmaceutique peut être administrée en association avec un anti-obésité tel que l'orlistat ou la sibutramine.

La composition pharmaceutique peut être administrée en association avec un composé utile dans le traitement ou la prévention des états pathologiques liés à des désordres lipidiques de la peau et aux complications inflammatoires et microbiennes ou des dérèglements de la production et/ou de la sécrétion de sébum associés à une hyperandrogénie (quelle qu'en soit l'origine = iatrogène, surrénaliennes, ou ovariennes), tel que les rétinoïdes, les antibiotiques, les antibactériens, ou les anti-androgènes.

Les rétinoïdes sont des dérivés de vitamine A utilisés habituellement pour le traitement de maladies dermatologiques.

On peut en particulier citer en tant que rétinoïdes : le rétinol, le rétinal, la trétinoïne, l'isotrétinoïne, l'alitrétinoïne, l'étretinate et son métabolite l'acitrétine, le tazarotène, le bexarotène ou l'adapalène.

Par antibiotiques, on entend ceux, par exemple, ayant pour cible la bactérie *Propionibacterium acnes* qui est impliquée dans certaines maladies dermatologiques telles que l'acné. Il peut s'agir d'antibiotiques à usage local comme par exemple la clindamycine ou l'érythromycine. Il peut s'agir également d'antibiotiques par voie orale tels que la doxycycline, la minocycline ou la tétracycline.

Les antimicrobiens sont ceux utilisés habituellement pour le traitement de certaines maladies dermatologiques tels que le peroxyde de benzoyl, ou l'acide azélaïque.

Les anti-androgènes pouvant être associés sont par exemple la progéstérone, l'estrogène, le finastéride, le dutastéride, la cyprotérone en association ou non avec l'éthinylestradiol, le flutamide, le nilutamide, ou le bicalutamide.

La composition pharmaceutique peut être administrée en association avec un composé utile dans le traitement ou la prévention du cancer en association avec d'autres traitements anticancéreux, qu'ils soient cytotoxiques et/ou cytostatiques, tels que les dérivés du platine, les taxanes, les vincas, le 5-FU, pour augmenter l'efficacité thérapeutique en vue du traitement des tumeurs réfractaires aux thérapeutiques usuelles.

La composition pharmaceutique selon l'invention peut être administrée par voie parentérale, par voie orale, par voie rectale ou par voie topique. Avantageusement, la composition pharmaceutique selon l'invention est administrée par voie topique.

Les compositions pharmaceutiques pour administration parentérale sont stériles et peuvent se présenter sous la forme de solutions aqueuses ou non aqueuses, de suspensions ou d'émulsions.

Les compositions pharmaceutiques pour administration orale peuvent être sous forme solide ou liquide. Comme compositions solides pour administration orale, on peut citer par exemple, les comprimés, les pilules, les poudres (capsules de gélatine, cachets) ou des granulés.
Comme compositions liquides pour administration orale, on peut citer les solutions, les suspensions, les émulsions et les sirops.

Les compositions pharmaceutiques pour administration par voie rectale sont par exemple les suppositoires ou les capsules rectales.

Les compositions pharmaceutiques pour administration par voie topique peuvent se présenter sous la forme de produits liquides (solutions, suspensions), de produits semi-solides (crème, gel, pommade, pâte, emplâtre, shampooing, mousse, lait, sérum, masque), ou de produits solides (poudre, stick solide) et peuvent éventuellement être conditionnées sous forme d'un aérosol ou d'un spray.

### SYNTHESE

Les composés de la présente invention peuvent être synthétisés en utilisant les voies de synthèse décrites ci-dessous ou en utilisant des méthodes de synthèse connues de l'homme de métier.

### Méthode 1

La synthèse des composés de formule générale I est caractérisée (schéma 1) en ce que l'on condense un dérivé de formule générale II 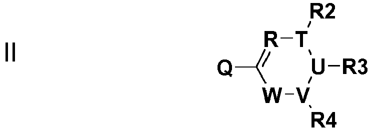 pour lequel :
Q représente Cl lorsque R représente N, T-U représente C=C, V représente N,
W représente C=O, R₂, R₃ et R₄ sont, dans ce cas là, tels que décrits précédemment dans la formule générale I,
Ou Q représente CH₃S ou CH₃S(O)₂ lorsque R représente N, T-U représente N=C, V-W représente C=N, R₂ ne représente pas de substituant, et R₃ et R₄ sont, dans ce cas là, tels que décrits précédemment dans la formule générale I,
Ou Q représente l'iode lorsque R représente C, TR₂ représente C=O, U représente N, R₃ est, dans ce cas là, tel que décrit précédemment dans la formule générale I, V-W représente N=CH et R₄ ne représente pas de substituant,
Ou Q représente CF₃S(O)₂O lorsque R représente C, T-U représente C=N, V représente N, W représente C=O, R₃ ne représente pas de substituant et R₂ et
R₄ sont, dans ce cas là, tels que décrits précédemment dans la formule générale I,
avec un dérivé de formule générale III 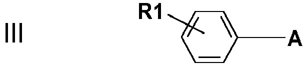 où A et R₁, sont tels que décrits précédemment dans la formule générale I. Cette réaction peut être effectuée en l'absence de base dans des solvants tels que le tétrahydrofurane ou l'éthanol (sous micro-ondes) ou en présence d'une base telle que la triéthylamine dans des solvants tels que le n-butanol ou l'acétonitrile;

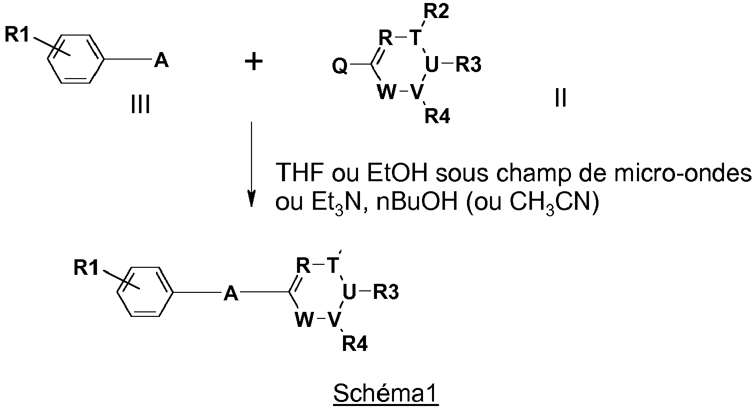

### Méthode 2

Cette méthode de synthèse des composés de formule générale I (schéma 2) est caractérisée en ce que l'on condense un dérivé de formule générale IV. 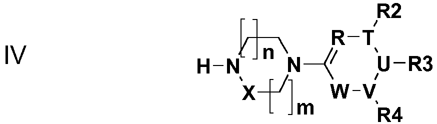 pour lequel X, n, m, R, T, U, V, W, R₂, R₃ et R₄ représentent les groupements tels que décrits précédemment dans la formule générale I avec un dérivé de formule générale V. 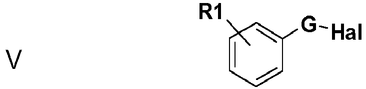 pour lequel Hal représente un halogène tel que Cl ou Br, G représente C=O ou -CH₂- et R₁ est tel que décrit précédemment dans la formule générale I.

Cette réaction peut être effectuée en présence de base telle que la triéthylamine ou la diisopropyléthylamine dans des solvants tels que le dichlorométhane ou le toluène (en particulier lorsque G représente -CH₂-) :

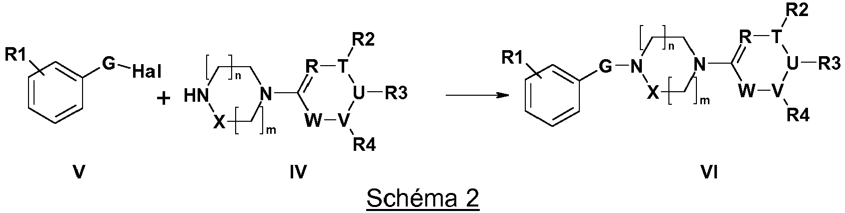

### Méthode 3

Cette méthode de synthèse des composés de formule générale I est caractérisée (schéma 3) en ce que l'on condense un dérivé de formule générale VII 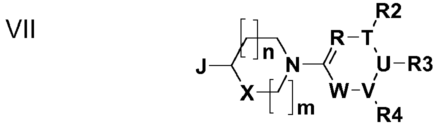 pour lequel J représente OH ou NH₂, et X, n, m, R, T, U, V, W, R₂, R₃ et R₄ sont tels que définis précédemment dans la formule générale I avec un dérivé de formule générale VIII : 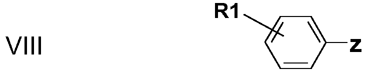 pour lequel R₁ est tel que défini dans la formule générale I et Z représente OH ou Br.

Cette réaction peut être effectuée dans des conditions opératoires telles que celles du couplage de Mitsunobu en présence triphénylphosphine, de diisopropylazodicarboxylate dans le THF (lorsque J et Z représentent OH) et dans des conditions opératoires telles que de couplage de Buchwald en présence de bis(diphénylphosphino)-1,1'-binaphtyl, de dipalladium bis[dibenzylidèneacétone], de tert-butoxyde de sodium dans le toluène (lorsque J= NH₂ et Z=Br).

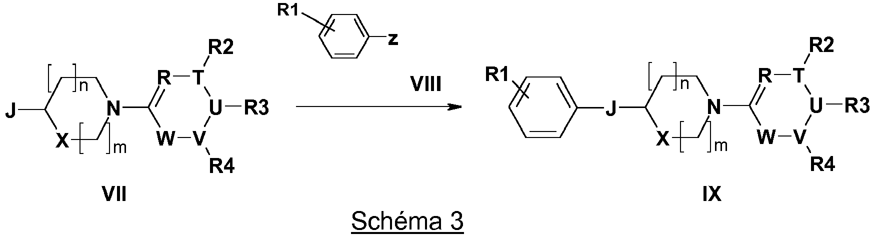

### Méthode 4

Cette méthode de synthèse des composés de formule générale I est caractérisée (schéma 4) en ce que l'on déshalogène la position 6 des composés de formule X pour lesquels R₂ représente Br ou Cl, R et V représentent un azote, T-U représente C=C, W représente C=O, R₁ représente trifluorométhyle, halogène tel que F, Cl, alkyle linéaire ou branché en C₁-C₄, trifluorométhoxy, acétyle, et R₃, R₄, D, E, X, n, m, sont tels que décrits précédemment dans la formule générale I.

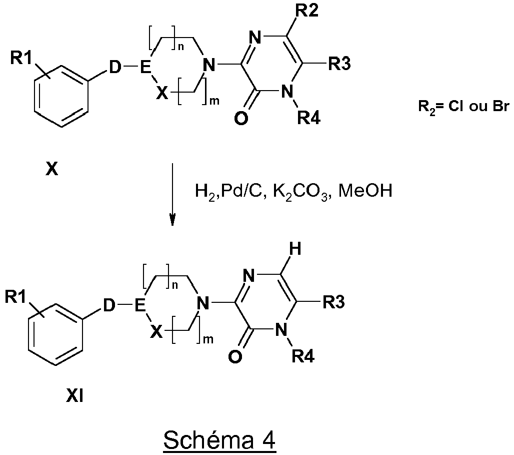

Les conditions opératoires sont telles que sous pression d'hydrogène en présence de palladium activé sur charbon et de carbonate de potassium dans le méthanol.

Les composés intermédiaires et finaux peuvent être, si on le désire, purifiés suivant une ou plusieurs méthodes de purification choisies parmi, l'extraction, la filtration, la chromatographie sur gel de silice, l'HPLC préparative sur phase normale ou inverse, la cristallisation.

Les matières premières utilisées dans les procédés décrits précédemment sont commerciales ou aisément accessibles à l'homme de métier selon des procédés décrits dans la littérature.

Les exemples suivants illustrent l'invention sans en limiter la portée.

Les analyses élémentaires et les spectres de masse et RMN confirment les structures des composés.

### INTERMEDIAIRES

### Intermédiaires 1:

### a) Chlorhydrate de la 4-(2-trifluorométhyl-phénoxy)-pipéridine (1a)

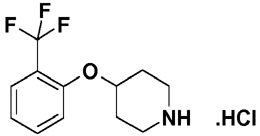

21,6g (107mmoles) de BOC-4-hydroxy-pipéridine sont placés en présence de 19,1g (118mmoles) de 2-trifluorométhylphénol, 33,8g (128mmoles) de triphénylphosphine dans 300mL de THF. A 0°C, 24,3mL (128mmoles) de DEAD sont ajoutés goutte-à-goutte. Le milieu réactionnel est agité pendant une heure à température ambiante puis chauffé pendant 24h à 70°C. Après concentration, le résidu obtenu est repris à l'éther, lavé avec une solution de soude (1N) puis avec une solution saturée en NaCl. Après séchage sur Na₂SO₄, les phases organiques sont concentrées à sec, puis reprises avec un mélange éther de pétrole-Et₂O: 70-30 pour éliminer l'oxyde de triphénylphosphine. Après filtration, le filtrat est concentré, le résidu obtenu est purifié par chromatographie flash sur silice (éther de pétrole-AcOEt, gradient 100-0 à 80-20 sur 50 min). 17,8g d'huile claire sont obtenus (rendement 48%). *CCM gel de silice 60 F 254 Merck, Ether de pétrole-AcOEt :90-10, Rf=0*,*26*. Cette huile est placée dans 300mL de dichlorométhane en présence de 23mL (309mmoles) de TFA, puis cette solution est agitée pendant 24h à température ambiante. Le milieu est concentré, le résidu obtenu est repris à l'AcOEt, lavé avec une solution aqueuse de soude (1N), puis à l'eau saturée en NaCl. Après séchage sur Na₂SO₄, les phases organiques sont concentrées à sec. 11,9g d'huile claire sont obtenus (rendement 94%). Cette huile est solubilisée dans le minimum d'EtOH puis traitée par 9mL d'une solution d'HCl (5N dans l'iPrOH). Après agitation à température ambiante pendant 3h, le précipité est filtré, rincé à l'éther éthylique puis séché. 9,6g d'intermédiaire 1a sont ainsi obtenus sous forme de solide blanc (rendement 70%). CCM gel de silice 60 F 254 Merck, CH₂Cl₂-MeOH-NH₄OH : 90-9-1, Rf=0,26.

### b) Intermédiaires 1b-1d

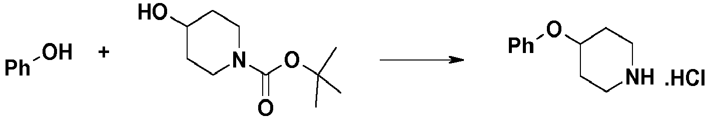

La synthèse des intermédiaires 1b-1d est réalisée suivant le mode opératoire décrit pour la synthèse de 1a en utilisant divers phénols Ph-OH.

**Tableau 1 : Intermédiaires 1b-1 d**

| PhOH | Rdt total | CCM | Etat | Intermédiaires 1b-1d |
|---|---|---|---|---|
| 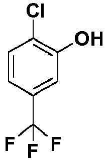 | 79% | CH₂Cl₂-MeOH-NH₄OH: 95-4.5-0.5 Rf=0,37 | solide | 1b: Chlorhydrate de la 4-(2-chloro-5-trifluorométhyl-phénoxy)-pipéridine |
| 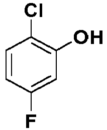 | 70% | CH₂Cl₂-MeOH-NH₄OH: 90-9-1 Rf=0,24 | solide | 1 c: Chlorhydrate de la 4-(2-chloro-5-fluoro-phénoxy)-pipéridine |
| 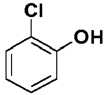 | 44% | CH₂Cl₂-MeOH-NH₄OH: 90-9-1 Rf=0,26 | solide | 1 d: Chlorhydrate de la 4-(2-chloro-phénoxy)-pipéridine |

| | | | | |
|---|---|---|---|---|
| CCM : gel de silice 60 F 254 Merck. | | | | |

### c) Chlorhydrate de la 3-(2-chloro-5-trifluorométhyl-phénoxyméthyl)-azétidine (1e)

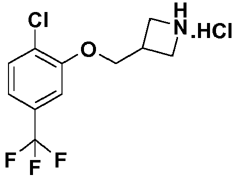

3g (11,84mmoles) de diphénylméthyl-3-(hydroxyméthyl)azétidine sont placés en présence de 2,55g (13,02mmoles) de 2-chloro-5-(trifluorométhyl)phénol et de 3,72g (14,20mmoles) de triphénylphosphine dans 70mL de THF. A 0°C, 2,23mL (14,20mmoles) de DEAD sont ajoutés goutte-à-goutte. Le milieu réactionnel est agité pendant une heure à température ambiante puis chauffé pendant 24h à 70°C. Après concentration, le résidu obtenu est repris au CH₂Cl₂ et lavé avec une solution de soude (1 N). Après séchage sur MgSO₄, les phases organiques sont concentrées à sec et le résidu obtenu est purifié par chromatographie flash sur silice (éther de pétrole-AcOEt, gradient 100-0 à 85-15 sur 50 min). 5g d'huile jaune sont obtenus (rendement 97%). *CCM gel de silice 60 F 254 Merck, Ether de pétrole-AcOEt : 80-20, Rf=0,39.* Cette huile est placée dans 75mL de 1,2-dichloroéthane en présence de 3,71mL (34,44mmoles) de 1-chloroéthylchloroformate puis cette solution est agitée pendant 20h à 70°C. 75mL de MeOH sont ensuite ajoutés et le milieu réactionnel est agité pendant 24h à 70°C. Après concentration à sec, le résidu obtenu est trituré dans de l'éther de pétrole, filtré puis rincé à l'éther de pétrole. Le solide obtenu est repris dans de l'eau et traité par du NaHCO₃ puis extrait au CH₂Cl₂. Après séchage sur MgSO₄, les phases organiques sont concentrées à sec et le résidu obtenu est purifié par chromatographie flash sur silice (CH₂Cl₂-MeOH-NH₄OH, gradient 100-0-0 à 90-9-1 sur 50 min). 0,69g de solide beige sont obtenus. Ce solide est solubilisé dans le minimum d'EtOH puis traité par 0,52mL d'une solution d'HCl (5N dans l'iPrOH). Après agitation à température ambiante pendant 3h, le précipité est filtré, rincé à l'éther éthylique puis séché. 0,691g d'intermédiaire 1e sont ainsi obtenus sous forme de solide blanc (rendement 20%). CCM gel de silice 60 F 254 Merck, CH₂Cl₂-MeOH-NH₄OH : 90-9-1, Rf=0,15.

### d) chlorhydrate de la trans 2-(2-chloro-5-trifluorométhyl-phénoxyméthyl)-cyclopropylamine (1f)

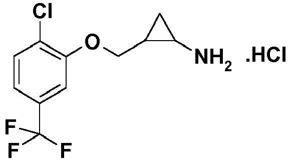

La synthèse de l'intermédiaire 1f est réalisée suivant le mode opératoire décrit pour la synthèse de 1a à partir du 2-chloro-5-(trifluorométhyl)phénol et de l'ester tert-butylique de l'acide trans-(2-hydroxyméthyl-cyclopropyl)-carbamique et, l'étape de déprotection de la fonction amine est réalisée dans une solution d'HCl (4N dans le dioxane). 1f est isolé sous forme de solide avec un rendement de 95%.
CCM gel de silice 60 F 254 Merck, CH₂Cl₂-MeOH-NH₄OH : 90-9-1, Rf=0,2.

### e) 4-o-Tolyloxy-pipéridine (1g)

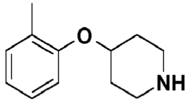

9g (44,7mmoles) de BOC-4-hydroxy-pipéridine sont placés dans 30mL de dichlorométhane à 0°C. 3,5 mL (44,7mmoles) de chlorure de mésyle et 8 mL (58,1mmoles) de triéthylamine sont ajoutés lentement. Le milieu réactionnel est agité pendant 3h à 0°C, puis filtré sur fritté. Le filtrat est lavé à l'eau. Après séchage sur MgSO₄, la phase organique est concentrée à sec. 12,48g d'huile sont obtenus (rendement quantitatif). *CCM gel de silice 60 F 254 Merck, CH₂Cl₂-MeOH :95-5, Rf=0,60.* 1,81 g (6,47mmoles) de cette huile sont placés en présence de 0,67 mL (6,47 mmoles) de 2-méthylphénol et de 4,09g (12,5mmoles) de carbonate de césium dans 10mL de DMF. Cette solution est agitée 24h à 70°C. Après concentration du milieu réactionnel, le résidu obtenu est purifié par chromatographie flash (éther de pétrole-AcOEt, gradient 100-0 à 85-15 sur 60 min). 0,9g d'huile claire est obtenu (rendement :48%). *CCM gel de silice 60 F 254 Merck, Ether de pétrole-AcOEt :95-5, Rf=0,28.* Cette huile est placée dans 5 mL de CH₂Cl₂ en présence de 0,58 mL (7,81 mmoles) de TFA. La solution est agitée pendant 6h à température ambiante. Après concentration du milieu réactionnel, le résidu est repris à l'AcOEt, lavé avec une solution de soude (1 N), puis à l'eau saturée en NaCl. Après séchage sur MgSO₄, la phase organique est concentrée à sec et 0,46g d'intermédiaire 1g sous forme d'une huile claire est obtenu (rendement 62%).
CCM gel de silice 60 F 254 Merck, CH₂Cl₂-MeOH :95-5, Rf=0,17.

### f) Chlorhydrate de la (2-trifluorométhyl-phényl)-pipérazin-1-yl-méthanone (1h)

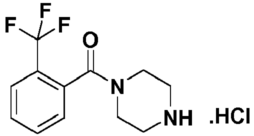

16,20g (86,90mmoles) de BOC-pipérazine sont placés dans 120mL de CH₂Cl₂ et 23mL (165mmoles) d'Et₃N sous azote. A 0°C, 17,27g (82,80mmoles) de chlorure de (2-trifluorométhyl)-benzoyle sont ajoutés goutte-à-goutte et le milieu réactionnel est agité pendant 30min à 0°C puis pendant 2h à température ambiante. Après concentration à sec, le résidu obtenu est repris dans de l'eau et extrait à l'AcOEt. Après séchage sur Na₂SO₄, les phases organiques sont concentrées à sec et le résidu obtenu est trituré dans de l'éther de pétrole puis filtré, rincé et séché sous vide. 28,5g de solide beige sont obtenus (rendement 96%). *CCM gel de silice 60 F 254 Merck, hexane-AcOEt : 50-50, Rif=0,24.* Ce solide est placé en présence de 100mL d'une solution d'HCl (5N dans l'iPrOH) dans 60mL d'EtOH et le milieu réactionnel est agité pendant 2h à 65°C. Après concentration à sec le résidu obtenu est trituré dans 200mL d'éther diéthylique puis filtré, rincé et séché sous vide. 22,6g d'intermédiaire 1 h sont ainsi obtenus sous forme de solide blanc (rendement 96%). CCM gel de silice 60 F 254 Merck, CH₂Cl₂-MeOH-NH₄OH : 90-9-1, Rf=0,34.

### Intermédiaires 2

### a) 3-Méthylsulfanyl-[1,2,4]triazine (2a)

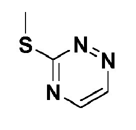

20g (219mmoles) de thiosemicarbazide sont placés dans 100mL d'éthanol. A 0°C, 13,8mL (219mmoles) d'iodométhane sont ajoutés goutte-à-goutte. Le mélange est chauffé pendant 4h à 80°C. Après refroidissement, le précipité formé est filtré, puis lavé à l'éther de pétrole. 48,63g de poudre jaune sont obtenus (rendement : 95%). *CCM gel de silice 60 F 254 Merck, CH₂Cl₂*-*AcOEt :90-10, Rf=0,18.* 15g (64,35mmoles) de ce solide sont placés dans 100mL d'eau glacée. Une solution de 50g (64,35mmoles) d'hydrogénocarbonate de sodium dans 50mL d'eau est additionnée. A 0°C, 9,3mL (64,35mmoles) de glyoxal sont additionnés goutte à goutte. Le milieu réactionnel est agité 18h à température ambiante. Après extraction du milieu réactionnel au dichlorométhane, les phases organiques sont séchées sur MgSO₄, puis concentrées à sec. 7,23g d'intermédiaire 2a sont obtenus sous forme d'huile jaune (rendement : 88%).
CCM gel de silice 60 F 254 Merck, CH₂Cl₂-AcOEt :95-5, Rf=0,4.

### b) intermédiaires 2b-2i

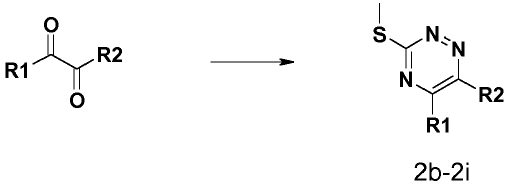

La synthèse des intermédiaires 2b-2i est réalisée suivant le mode opératoire décrit pour la synthèse de 2a en utilisant divers dérivés du glyoxal dans l'eau à température ambiante ou dans l'éthanol à reflux.

**Tableau 2 : intermédiaires 2b-2i**

| R1(C=O)₂R2 | Rdt | CCM | Etat | intermédiaires 2b-2i |
|---|---|---|---|---|
| 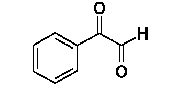 | 92% | CH₂Cl₂-MeOH:95-5 Rf=0,55 | solide | 2b : 3-Méthylsulfanyl-5-phényl-[1,-2,4]triazine |
| 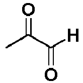 | 60% | CH₂Cl₂-AcOEt:95-5 Rf=0,41 | solide | 2c : 5-Méthyl-3-méthylsulfanyl-[1,2,4]triazine |
| 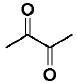 | 100% | CH₂Cl₂-MeOH:95-5 Rf=0,64 | solide | 2d: 5,6-Diméthyl-3-méthylsulfanyl-[1,2, 4]triazine |
| 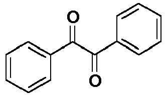 | 100% | CH₂Cl₂-AcOEt:95-5 Rf=0,59 | solide | 2e: 3-Méthylsulfanyl-5,6-diphényl-[1,2, 4]triazine |
| 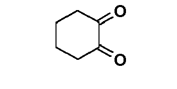 | 95% | CH₂Cl₂-AcOEt:80-20 Rf=0,59 | solide | 2f : 3-Méthylsulfanyl-5,6,7,8-tétrahydro-benzo[1,2,4]triazine |
| 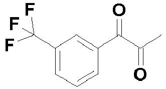 | 39% | CH₂Cl₂-AcOEt:90-10 Rf=0,68 | solide | 2g : 6-Méthyl-3-méthylsulfanyl-5-(3-trifluorométhyl-phényl)-[1,2,4]-triazine |
| 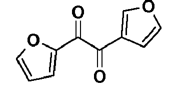 | 100% | CH₂Cl₂-AcOEt:95-5 Rf=0,68 | solide | 2h : 5,6-Di-furan-3-yl-3-méthylsul-fanyl-[1,2,4]triazine |
| 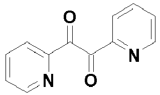 | 92% | CH₂Cl₂-MeOH:95-5 Rf=0,67 | solide | 2i : 3-Méthylsulfanyl-5,6-di-pyridin-2-yl-[1,2,4]triazine |

| | | | | |
|---|---|---|---|---|
| CCM : gel de silice 60 F 254 Merck. | | | | |

### c) 6-Méthyl-3-méthylsulfanyl-[1,2,4]triazine (2j)

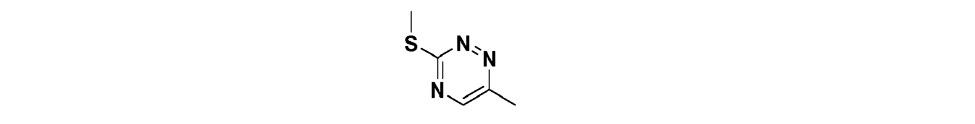

20g (219mmoles) de thiosemicarbazide sont placés dans 100mL d'éthanol. A 0°C, 13,8mL (219mmoles) d'iodométhane sont ajoutés goutte-à-goutte. Le mélange est chauffé pendant 4h à 80°C. Après refroidissement, le milieu est filtré, le précipité formé est lavé à l'éther de pétrole. 48,63g de poudre jaune sont obtenus (rendement : 95%). *CCM gel de silice 60 F 254 Merck, CH₂Cl₂*-*AcOEt :90-10, Rf=0,18.* 4g (17,16mmoles) de ce solide sont placés dans 25mL de méthanol. 2,05mL (17,16mmoles) d'aldéhyde pyruvique-diméthylacétal sont additionnés goutte à goutte. Le milieu réactionnel est chauffé pendant 4h à 70°C. Après concentration, le résidu obtenu est repris avec une solution saturée en NaCl et extrait à l'acétate d'éthyle. Après séchage sur MgSO₄, les phases organiques sont concentrées à sec. Le résidu obtenu est purifié par chromatographie flash (gradient CH₂Cl₂-AcOEt : 100-0 à 90-10). 1,11g d'intermédiaire 2j sont obtenus sous forme de solide jaune (rendement : 46%).
CCM gel de silice 60 F 254 Merck, CH₂Cl₂-AcOEt :90-10, Rf=0,34.

### Intermédiaires 3

### a) 3-Méthanesulfonyl-5,6-diméthyl-[1,2,4]triazine (3a)

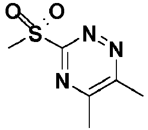

3,57g (23mmoles) d'intermédiaire 2d sont placés dans 90mL de dichlorométhane. A 0°C, 11,3g (46mmoles) d'acide méta-chloroperbenzoïque sont ajoutés par portions. Le mélange est agité à température ambiante pendant 2h. Après filtration du précipité, le filtrat est concentré, le résidu est purifié par chromatographie flash (Ether de pétrole 100 pendant 6 min, puis gradient CH₂Cl₂-AcOEt : 100-0 à 20-80 sur 40min). 3,6g d'intermédiaire 3a sont obtenus sous forme de solide jaune (rendement : 83%).
CCM gel de silice 60 F 254 Merck, CH₂Cl₂-AcOEt: 90-10, Rf=0,31.

### b) Intermédiaires 3b-3g

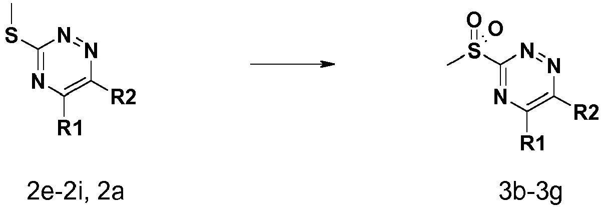

La synthèse des intermédiaires 3b-3g est réalisée respectivement à partir des composés 2e-2i, 2a suivant le mode opératoire décrit pour la synthèse de 3a.

**Tableau 3 : intermédiaires 3b-3g**

| Synthons de départ | Rdt | CCM | Etat | intermédiaires 3b-3g |
|---|---|---|---|---|
| 2e | 75% | CH₂Cl₂-AcOEt:90-10 Rf=0,56 | solide | 3b: 3-Méthanesulfonyl-5,6-diphényl-[1,2,4]triazine |
| 2f | 43% | CH₂Cl₂-AcOEt: 80-20 Rf=0,42 | solide | 3c: 3-Méthanesulfonyl-5,6,7,8-tétrahydro-benzo[1,2,4]triazine |
| 2g | 94% | CH₂Cl₂-AcOEt: 90-10 Rf=0,68 | solide | 3d: 3-Méthanesulfonyl-6-méthyl-5-(3-trifluorométhyl-phényl)-[1,2,4]triazine |
| 2h | 53% | CH₂Cl₂-AcOEt: 75-25 Rf=0,80 | solide | 3e: 5,6-Di-furan-2-yl-3-méthanesulfonyl-[1,2,4]triazine |
| 2i | 35% | CH₂Cl₂-MeOH:95-5 Rf=0,37 | solide | 3f: 3-Méthanesulfonyl-5,6-di-pyridin-2-yl-[1,2,4]triazine |
| 2a | 18% | CH₂Cl₂-AcOEt: 95-5 Rf=0,23 | solide | 3g: 3-Méthanesulfonyl-[1,2,4]triazine |

### Intermédiaires 4

### a) 5-Iodo-2-méthyl-2H-pyridazin-3-one (4a)

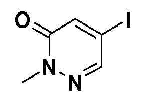

1,55g (8,65mmoles) de 4,5-dichloro-2-méthyl-2H-pyridazin-3-one sont placés dans 50mL d'une solution aqueuse d'acide iodidrique à 57%. Le mélange est chauffé pendant 24h à 137°C. Après retour à température ambiante, le milieu réactionnel est versé sur une solution aqueuse de thiosulfate de sodium (20g dans 250mL d'eau). Le mélange est repris au dichlorométhane, lavé à l'eau, puis avec une solution saturée en NaCl. Après séchage sur Na₂SO₄, la phase organique est concentrée à sec. Le résidu obtenu est trituré dans un mélange dichlorométhane-méthanol 50-50, le précipité est isolé par filtration. 3,45g d'intermédiaire 4a sont obtenus sous forme de poudre jaune (rendement : quantitatif).
CCM gel de silice 60 F 254 Merck, CH₂Cl₂-AcOEt :70-30, Rf=0,4.

### b) Ester 2-méthyl-3-oxo-2,3-dihydro-pyridazin-4-yl de l'acide trifluoro-méthanesulfonique (4b)

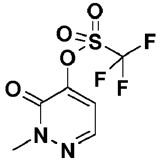

3,13g (27,93mmoles) de tertbutylate de potassium sont placés dans 25mLde tétrahydrofurane. A 0°C est ajoutée une solution de 1,16mL (27,93mmoles) de méthanol dans 10mLde tétrahydrofurane. Le mélange est agité à 0°c pendant 10min. Cette solution est additionnée goutte à goutte à un mélange refroidi à 0°C de 5g (27,93mmoles) de 4,5-dichloro-2-méthyl-2H-pyridazin-3-one solubilisés dans 40mL de tétrahydrofurane, la température du milieu reste inférieure à 3°C pendant l'addition. Le mélange est agité pendant 1 h à 0°C, puis pendant 3h à température ambiante. Le milieu est repris au dichlorométhane et lavé à l'eau. Après séchage sur Na₂SO₄, la phase organique est concentrée à sec. Le résidu obtenu est purifié par chromatographie flash (CH₂Cl₂-AcOEt : 95-5). 4,45g de solide blanc sont obtenus (rendement : 91%). *CCM gel de silice 60 F 254 Merck, CH₂Cl₂-AcOEt :90-10, Rf=0,55.* 3,37g (19,3mmoles) de ce solide sont placés dans 150mL de tétrahydrofurane en présence 2,7mL (19,3mmoles) de triéthylamine et de 0,67g de palladium sur charbon à 10%. Le milieu est mis sous pression d'hydrogène (7bars) et laissé sous agitation à température ambiante pendant 48h. Après filtration sur célite du milieu réactionnel, le filtrat est concentré. Le résidu obtenu est purifié par chromatographie flash (gradient CH₂Cl₂-AcOEt : 90-10 à 10-90). 2,35g de solide blanc sont obtenus (rendement : 86%). *CCM gel de silice 60 F 254 Merck, CH₂Cl₂-AcOEt :50-50, Rf=0,17.* 2,35g (16,7mmoles) de ce solide sont placés dans 250mL d'eau en présence de 9,6g (16,7mmoles) d'hydroxyde de potassium. Le mélange est chauffé à 100°C pendant 24h. Le milieu refroidi à 0°C est amené à pH 1-2 par addition d'une solution aqueuse d'acide chlorhydrique concentré. Après concentration à sec, le résidu est repris avec un mélange dichlorométhane / méthanol, les minéraux sont éliminés par filtration et le filtrat est concentré à sec. Le résidu obtenu est purifié par chromatographie flash (CH₂Cl₂-MeOH : 95-5). 1,94g de solide rose sont obtenus (rendement : 92%). *CCM gel de silice 60 F 254 Merck, CH₂Cl₂-MeOH :80-20, Rf=0,49.* 1g (7,92mmoles) de ce solide est placé sous azote dans 15mL de dichlorométhane. A -9°C, sont additionnés 1,45mL (10,3mmoles) de triéthylamine, puis 1,8mL (10,7mmoles) d'anhydride trifluorométhanesulfonique. Après agitation 20min à -7°C, 5mL d'une solution aqueuse d'acide chlorhydrique 1 N sont ajoutés. La phase organique est lavée à l'eau, puis avec une solution aqueuse à 1% en bicarbonate de sodium, puis avec une solution saturée en NaCl. Après séchage sur Na₂SO₄, les phases organiques sont concentrées à sec. 1,9g d'intermédiaire 4b sont obtenus sous forme de solide rose (rendement : 93%).
CCM gel de silice 60 F 254 Merck, CH₂Cl₂-MeOH :95-5, Rf=0,78.

### d) 3,5-Dichloro-1-méthyl-1H-pyrazin-2-one (4c)

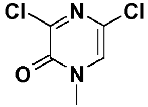

3,29g (30,8mmoles) de chlorhydrate du méthylamino-acétonitrile sont placés en présence de 19,6g (154mmoles) de chlorure d'oxalyle dans 30mL de 1,2-dichlorobenzène. Le mélange est chauffé pendant 8h à 80°C. Après concentration à sec du milieu réactionnel, le résidu obtenu est purifié par chromatographie flash (gradient Ether de pétrole-CH₂Cl₂ 100-0 à 0-100). 3,35g d'intermédiaire 4c sont obtenus sous forme de poudre beige (rendement : 60%).
CCM gel de silice 60 F 254 Merck, CH₂Cl₂-MeOH :90-10, Rf=0,79.

### Intermédiaires 5

### a) 3-Pipérazin-1-yl-[1,2,4]triazine (5a)

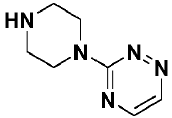

3,45g (40,1 mmoles) de pipérazine sont placés en présence de 1,7g (13,4mmoles) d'intermédiaire 2a dans le 1-butanol. 6,5mL (47mmoles) de triéthylamine sont ajoutés et le mélange est chauffé pendant 24h à 120°C. Après concentration du milieu réactionnel, le résidu obtenu est repris à l'acétate d'éthyle et lavé à l'eau. Après séchage sur MgSO₄, la phase organique est concentrée à sec. Le résidu obtenu est purifié par chromatographie flash (gradient CH₂Cl₂-MeOH-NH₄OH : 100-0-0 à 90-9-1). 1,52g d'intermédiaire 5a sont obtenus sous forme d'huile brune (rendement : 69%).
CCM gel de silice 60 F 254 Merck, CH₂Cl₂-MeOH-NH₄OH :90-9-1, Rf=0,26.

### b) 1-[1,2,4]Triazin-3-yl-pipéridin-4-ylamine (5b)

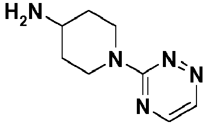

La synthèse de l'intermédiaire 5b est réalisée à partir de la pipéridin-4-ylamine et du précurseur 2a suivant le mode opératoire décrit pour la synthèse de 5a. L'intermédiaire 5b est isolé sous forme de solide avec un rendement de 71%. CCM gel de silice 60 F 254 Merck, CH₂Cl₂-MeOH :95-5, Rf=0,1.

### c) 3-Méthyl-1-[1,2,4]triazin-3-yl-pipéridin-4-ol (5c)

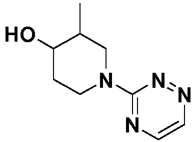

La synthèse de l'intermédiaire 5c est réalisée à partir du 3-méthyl-pipéridin-4-ol et du précurseur 2a suivant le mode opératoire décrit pour la synthèse de 5a. L'intermédiaire 5c est isolé sous forme de solide avec un rendement de 47%. CCM gel de silice 60 F 254 Merck, CH₂Cl₂-MeOH :95-5, Rf=0,4.

### d) 1-[1,2,4]Triazin-3-yl-pyrrolidin-3-ol (5d)

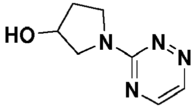

La synthèse de l'intermédiaire 5d est réalisée à partir du pyrrolidin-3-ol et du précurseur 2a suivant le mode opératoire décrit pour la synthèse de 5a. L'intermédiaire 5d est isolé sous forme d'huile avec un rendement de 92%. CCM gel de silice 60 F 254 Merck, CH₂Cl₂-MeOH :95-5, Rf=0,5.

### e) Intermédiaires 5e-5g

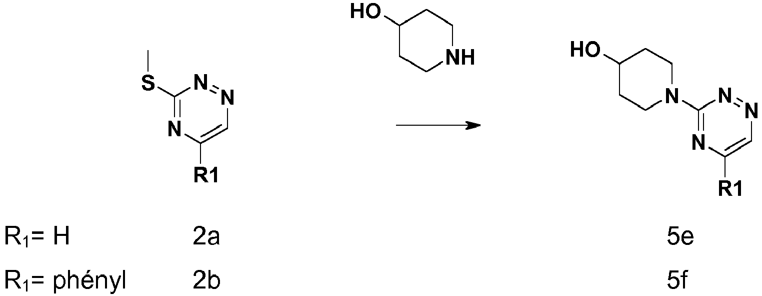

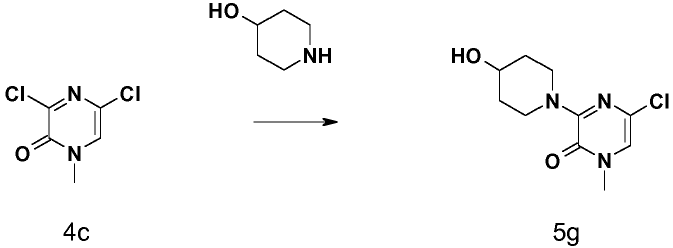

La synthèse des intermédiaires 5e-5g est réalisée respectivement à partir des composés 2a, 2b et 4c et de la pipéridin-4-ol suivant le mode opératoire décrit pour la synthèse de 5a.

**Tableau 4 : intermédiaires 5e-5g**

| Synthons de départ | Rdt | CCM | Etat | intermédiaires 5e-5g |
|---|---|---|---|---|
| 2a | 63% | CH₂Cl₂-MeOH-NH₄OH: 90-9-1 Rf=0,43 | solide | 5e: 1-[1,2,4]Triazin-3-yl-pipéridin-4-ol |
| 2b | 84% | CH₂Cl₂-MeOH:95-5 Rf=0,26 | solide | 5f : 1-(5-Phényl-[1,2,4]triazin-3-yl)-pipéridin-4-ol |
| 4c | 60% | CH₂Cl₂-MeOH:95-5 Rf=0,28 | solide | 5g : 5-Chloro-3-(4-hydroxy-pipéridin-1-yl)-1-méthyl-1H-pyrazin-2-one |

| | | | | |
|---|---|---|---|---|
| CCM gel de silice 60 F 254 Merck | | | | |

### EXEMPLES

### Exemple 1 (exemple de référence) : 6'-Chloro-4'-méthyl-4-(2-trifluorométhyl-benzoyl)-3,4,5,6-tétrahydro-2H,4'H-[1,2']bipyrazinyl-3'-one (1)

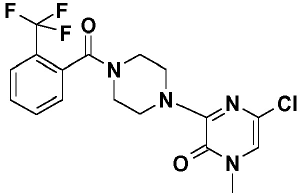

Le composé 1 est préparé selon la méthode de synthèse 1 : 1,76g (5,97mmoles) de dérivé 1 h et 1,13g (6,31 mmoles) de pyrazinone 4c sont placés dans 3 mL de butanol-1 en présence de 4mL (27,9mmoles) de NEt₃. Ce mélange est agité à 120°C pendant 24 h. Après concentration à sec du milieu réactionnel, le résidu obtenu est repris à l'AcOEt et lavé à l'eau et avec une solution saturée en NaCl. Après séchage sur MgSO₄, la phase organique est concentrée à sec. Le résidu obtenu est purifié par chromatographie flash sur silice (gradient CH₂Cl₂-AcOEt : 100-0 à 90-10). On isole 0,86g de solide beige (rendement: 36%).
CCM gel de silice 60 F 254 Merck, CH₂Cl₂-MeOH : 95-5, Rf=0,71.
F=162°C
RMN ¹H (CDCl₃) ppm: 3,28 (t, 2H, J=5,18Hz), 3,44 (s, 3H), 3,67-3,88 (m, 3H), 3,93-4,09 (m, 3H), 6,73 (s, 1 H), 7,35 (d, 1 H, J=7,6Hz), 7,54 (t, 1 H, J=7,6Hz), 7,62 (t, 1 H, J=7,6Hz), 7,73 (d, 1 H J=8Hz).
MS (+ESI) m/z 401 (MH+)

### Exemple 2 (exemple de référence) : 4'-Méthyl-4-(2-trifluorométhyl-benzoyl)-3,4,5,6-tétrahydro-2H,4'H-[1,2']bipyrazinyl-3'-one (2)

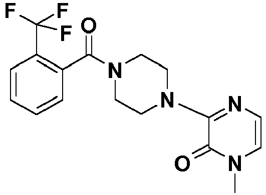

Le composé 2 est préparé selon la méthode de synthèse 4: 0,46g (1,15mmoles) du composé décrit pour l'exemple 1 sont placés dans 5mL de méthanol en présence de 0,15g (1,15mmoles) de carbonate de potassium et de 0,05g de palladium activé sur charbon (5%). Le milieu est mis sous pression d'hydrogène (7bars) et laissé sous agitation à température ambiante pendant 48h. Après filtration sur célite du milieu réactionnel, le filtrat est concentré. Le résidu obtenu est purifié par chromatographie flash (gradient CH₂Cl₂-MeOH : 100-0 à 98-2). 0,26g de solide beige sont obtenus (rendement : 62%).
CCM gel de silice 60 F 254 Merck, CH₂Cl₂-MeOH : 95-5, Rf=0,54.
F=142°C
RMN ¹H (CDCl₃) ppm: 3,28 (t, 2H, J=5,42Hz), 3,47 (s, 3H), 3,69 (t, 2H, J=5,42Hz), 3,81-4,00 (m, 4H), 6,69 (d, 1 H, J=4,42Hz), 6,91 (d, 1 H, J=4,42Hz), 7,35 (d, 1 H, J=7,6Hz), 7,54 (t, 1 H, J=7,6Hz), 7,61 (t, 1 H, J=7,6Hz), 7,72 (t, 1 H, J=8Hz).
MS (+ESI) m/z 367 (MH+)

### Exemple 3 (exemple de référence) : (4-[1,2,4]Triazin-3-yl-pipérazin-1-yl)-(2-trifluorométhyl-phényl)-méthanone (3)

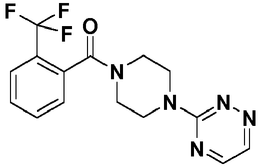

Le composé 3 est préparé selon la méthode de synthèse 2 : 0,4g (2,42mmoles) de dérivé 5a sont placés dans 10mL de CH₂Cl₂. 0,5mL (3,63mmoles) de triéthylamine sont additionnés. A 0°C, 0,4mL (2,66mmoles) de chlorure de trifluorométhylbenzoyle sont ajoutés goutte à goutte, le milieu réactionnel est agité à température ambiante pendant 18h. Après concentration à sec du milieu réactionnel, le résidu obtenu est repris à l'AcOEt et lavé à l'eau. Après séchage sur MgSO₄, la phase organique est concentrée. Le résidu obtenu est purifié par chromatographie flash sur silice (CH₂Cl₂:100%). On isole 0,72g de poudre jaune (rendement: 95%).
CCM gel de silice 60 F 254 Merck, CH₂Cl₂-MeOH-NH₄OH : 90-9-1, Rf=0,6.
F=104°C
RMN ¹H (CDCl₃) ppm: 3,30 (t, 2H, J=5,2Hz), 3,82-3,92 (m, 3H), 3,97-4,10 (m, 3H), 7,37 (d, 1 H, J=7,6Hz), 7,56 (t, 1 H, J=7,6Hz), 7,64 (t, 1 H, J=7,6Hz), 7,75 (d, 1 H, J=8Hz), 8,16 (d, 1 H, J= 2Hz), 8,57 (d, 1 H, J= 2,4Hz).
MS (+APCI) m/z 338 (MH+)

### Exemple 4 (exemple de référence): (5-Fluoro-2-trifluorométhyl-phényl)-(4-[1,2,4]triazin-3-yl-pipérazin-1-yl)-méthanone (4)

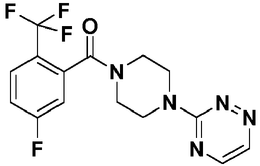

Le composé 4 est préparé à partir du chlorure du 5-fluoro-2-trifluorométhyl-benzoyle et de l'intermédiaire 5a selon la méthode de synthèse 2 dans les conditions opératoires décrites pour l'exemple 3 (rendement 67%).
CCM gel de silice 60 F 254 Merck, CH₂Cl₂-MeOH-NH₄OH : 90-9-1, Rf=0,77.
F=122°C
RMN ¹H (CDCl₃) ppm: 3,31 (t, 2H, J=5,24Hz), 3,83-3,92 (m, 5H), 3,95-4,07 (m, 1 H), 7,07-7,10 (m, 1 H), 7,22-7,23 (m, 1 H), 7,74-7,77 (m, 1 H), 8,16 (d, 1 H, J= 2,1 Hz), 8,57 (d, 1 H, J= 2,1 Hz).
MS (+ESI) m/z 356 (MH+)

### Exemple 5 (exemple de référence): 3-[4-(2-Trifluorométhyl-benzyl)-pipérazin-1-yl]-[1,2,4]triazine (5)

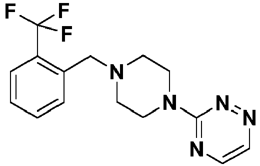

Le composé 5 est préparé à partir du 1-bromométhyl-2-trifluorométhyl-benzène et de l'intermédiaire 5a selon la méthode de synthèse 2 dans le toluène à reflux (rendement 33%).
CCM gel de silice 60 F 254 Merck, CH₂Cl₂-MeOH-NH₄OH : 90-9-1, Rf=0,63. RMN ¹H (CDCl₃) ppm: 2,54-2,59 (m, 4H), 3,91 (s, 2H), 3,93-3,95 (m, 4H), 7,36 (t, 1 H, J= 7,6Hz), 7,55 (t, 1 H, J= 7,6Hz), 7,64 (d, 1 H, J= 7,84Hz), 7,84 (d, 1 H, J= 7,76Hz), 8,11 (d, 1 H, J= 2,2Hz), 8,49 (d, 1 H, J= 2,2Hz).
MS (+ESI) m/z 324 (MH+)

### Exemple 6 : 5-Chloro-3-[4-(2-chloro-phénoxy)-pipéridin-1-yl]-1-méthyl-1H-pyrazin-2-one (6)

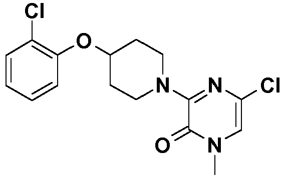

Le composé 6 est préparé à partir de la pyrazinone 4c et de l'intermédiaire 1d selon la méthode de synthèse 1 dans les conditions opératoires décrites pour l'exemple 1 (rendement 49%).
CCM gel de silice 60 F 254 Merck, CH₂Cl₂-MeOH : 95-5, Rf=0,69.
F=102°C
RMN ¹H (CDCl₃) ppm: 1,97-2,03 (m, 4H), 3,40 (s, 3H), 3,99-4,20 (m, 4H), 4,61 (m, 1 H), 6,66 (s, 1 H), 6,91 (t, 1 H, J=7,6Hz), 6,97 (d, 1 H, J=8,1 Hz), 7,20 (t, 1 H, J=7,6Hz), 7,37 (d, 1 H J=7,76Hz). MS (+ESI) m/z 354 (MH+)

### Exemple 7 : 5-Chloro-1-méthyl-3-[4-(2-trifluorométhyl-phénoxy)-pipéridin-1-yl]-1H-pyrazin-2-one (7)

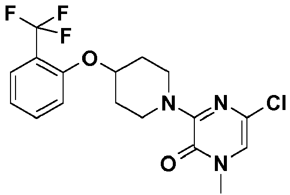

Le composé 7 est préparé à partir de la pyrazinone 4c et de l'intermédiaire 1a selon la méthode de synthèse 1 dans les conditions opératoires décrites pour l'exemple 1 (rendement 25%).
CCM gel de silice 60 F 254 Merck, CH₂Cl₂-AcOEt : 90-10, Rf=0,43.
F=90°C
RMN ¹H (CDCl₃) ppm: 1,96-2,07 (m, 4H), 3,42 (s, 3H), 3,89-3,94 (m, 2H), 4,12-4,17 (m, 2H), 4,72 (m, 1 H), 6,66 (s, 1 H), 6,99 (m, 2H), 7,47 (t, 1 H, J=7,84Hz), 7,58 (d, 1 H J=7,64Hz).
MS (+ESI) m/z 388 (MH+)

### Exemple 8 : 5-Chloro-3-[4-(2-chloro-5-trifluorométhyl-phénoxy)-pipéridin-1-yl]-1-méthyl-1H-pyrazin-2-one (8)

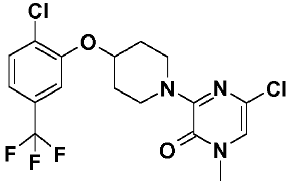

Le composé 8 est préparé selon la méthode de synthèse 3 : 0,2g (0,82mmoles) d'intermédiaire 5g sont placés en présence de 0,17g (0,90mmoles) de 2-chloro-5-trifluorométhylphénol, 0,26g (0,98mmoles) de triphénylphosphine dans 5mL de THF. A 0°C, 0,18mL (0,98mmoles) de DEAD sont ajoutés goutte-à-goutte. Le milieu réactionnel est chauffé pendant 8h à 70°C, puis laissé sous agitation à température ambiante pendant 15h. Après concentration, le résidu obtenu est repris à l'éther et lavé avec une solution de soude (1N). Après séchage sur MgSO₄, la phase organique est concentrée à sec, le résidu obtenu est purifié par chromatographie flash sur silice (gradient CH₂Cl₂-AcOEt : 100-0 à 95-5 sur 30 min). 0,11g de solide rosé sont obtenus (rendement 31%).
CCM gel de silice 60 F 254 Merck, CH₂Cl₂-AcOEt : 95-5, Rf=0,58.
F=111°C
RMN ¹H (CDCl₃) ppm: 1,94-2,04 (m, 4H), 3,43 (s, 3H), 4,03-4,07 (m, 4H), 4,68 (m, 1 H), 6,67 (s, 1 H), 7,17 (s, 1 H), 7,18 (d, 1 H, J=8,1 Hz), 7,49 (d, 1 H, J=8Hz).
MS (+ESI) m/z 422 (MH+)

### Exemple 9 : 5-Chloro-1-méthyl-3-(4-o-tolyloxy-pipéridin-1-yl)-1H-pyrazin-2-one (9)

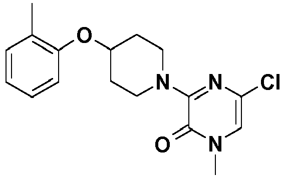

Le composé 9 est préparé à partir de la pyrazinone 4c et de l'intermédiaire 1g selon la méthode de synthèse 1 dans les conditions opératoires décrites pour l'exemple 1 (rendement 59%).
CCM gel de silice 60 F 254 Merck, CH₂Cl₂-AcOEt : 95-5, Rf=0,72.
F=102°C
RMN ¹H (CDCl₃) ppm: 1,89-1,96 (m, 2H), 2,01-2,08 (m, 2H), 2,24 (s, 3H), 3,42 (s, 3H), 3,93-3,99 (m, 2H), 4,03-4,11 (m, 2H), 4,54-4,57 (m, 1H), 6,66 (s, 1H), 6,84-6,88 (m, 2H), 7,12-7,16 (m, 2H).
MS (+APCI) m/z 334 (MH+)

### Exemple 10 : 1-Méthyl-3-(4-o-tolyloxy-pipéridin-1-yl)-1H-pyrazin-2-one (10)

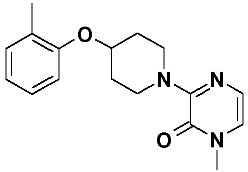

Le composé 10 est préparé à partir du dérivé décrit pour l'exemple 9 selon la méthode de synthèse 4 dans les conditions opératoires décrites pour l'exemple 2, en utilisant comme solvant un mélange (1/1) méthanol/ dichlorométhane (rendement 61 %).
CCM gel de silice 60 F 254 Merck, CH₂Cl₂-AcOEt : 95-5, Rf=0,28.
F=97°C
RMN ¹H (CDCl₃) ppm: 1,89-1,96 (m, 2H), 2,03-2,09 (m, 2H), 2,24 (s, 3H), 3,63 (s, 3H), 3,73-3,79 (m, 2H), 3,98-4,04 (m, 2H), 4,53-4,56 (m, 1 H), 6,63 (d, 1 H, J=4,4Hz), 6,84-6,87 (m, 2H), 6,91 (d, 1 H, J=4,4Hz), 7,12-7,16 (m, 2H).
MS (+ESI) m/z 300 (MH+)

### Exemple 11 : 1-Méthyl-3-[4-(2-trifluorométhyl-phénoxy)-pipéridin-1-yl]-1H-pyrazin-2-one (11)

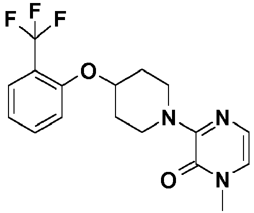

Le composé 11 est préparé à partir du dérivé décrit pour l'exemple 7 selon la méthode de synthèse 4 dans les conditions opératoires décrites pour l'exemple 2, en utilisant comme solvant un mélange (1/1) méthanol/ dichlorométhane (rendement 70%).
CCM gel de silice 60 F 254 Merck, CH₂Cl₂-AcOEt : 95-5, Rf=0,49.
F=90°C
RMN ¹H (CDCl₃) ppm: 1,94-2,10 (m, 4H), 3,46 (s, 3H), 3,86-3,96 (m, 4H), 4,68-4,73 (m, 1 H), 6,63 (d, 1 H, J=4,4Hz), 6,91 (d, 1 H, J=4,4Hz), 6,96-7,02 (m, 2H), 7,47 (t, 1 H, J= 7,6Hz), 7,58 (d, 1 H, J=7,6Hz).
MS (+ESI) m/z 354 (MH+)

### Exemple 12 : 5-Chloro-3-[4-(2-chloro-5-fluoro-phénoxy)-pipéridin-1-yl]-1-méthyl-1H-pyrazin-2-one (12)

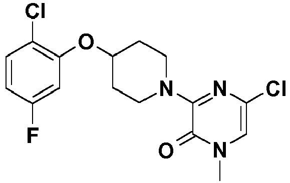

Le composé 12 est préparé à partir de la pyrazinone 4c et de l'intermédiaire 1 c selon la méthode de synthèse 1 dans les conditions opératoires décrites pour l'exemple 1 (rendement 38%).
CCM gel de silice 60 F 254 Merck, CH₂Cl₂-AcOEt : 95-5, Rf=0,67.
F=84°C
RMN ¹H (CDCl₃) ppm: 1,92-2,08 (m, 4H), 3,43 (s, 3H), 4,03-4,06 (m, 4H), 4,56-4,61 (m, 1 H), 6,67 (s, 1 H), 6,62-6,72 (m, 2H), 7,29-7,33 (m, 1 H).
MS (+APCI) m/z 372 (MH+)

### Exemple 13(exemple de référence) : 3-[4-(2-Chloro-phénoxy)-pipéridin-1-yl]-[1,2,4]triazine (13)

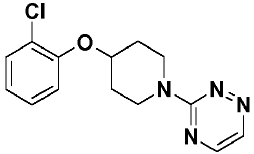

Le composé 13 est préparé à partir du 2-chlorophénol et de l'intermédiaire 5e selon la méthode de synthèse 3 dans les conditions opératoires décrites pour l'exemple 8 (rendement 50%).
CCM gel de silice 60 F 254 Merck, CH₂Cl₂-AcOEt : 95-5, Rf=0,25.
F=67°C
RMN ¹H (DMSO-d₆) ppm: 1,69-1,77 (m, 2H), 1,97-2,04 (m, 2H), 3,78-3,84 (m, 2H), 4,06-4,12 (m, 2H), 4,79-4,84 (m, 1 H), 6,95-7,00 (m, 1 H), 7,28-7,33 (m, 2H), 7,44 (d, 1 H, J= 8Hz), 8,63 (d, 1 H, J= 2,2Hz), 8,34 (d, 1 H, J= 2,2Hz).
MS (+ESI) m/z 291 (MH+)

### Exemple 14 (exemple de référence) : 3-[4-(2-Trifluorométhyl-phénoxy)-pipéridin-1-yl]-[1,2,4]triazine (14)

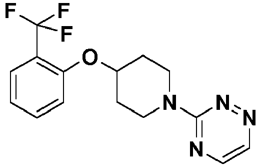

Le composé 14 est préparé à partir du 2-trifluorométhylphénol et de l'intermédiaire 5e selon la méthode de synthèse 3 dans les conditions opératoires décrites pour l'exemple 8 (rendement 30%).
CCM gel de silice 60 F 254 Merck, CH₂Cl₂-AcOEt : 95-5, Rf=0,33.
F=70°C
RMN ¹H (CDCl₃) ppm: 1,96-2,05 (m, 4H), 3,88-3,95 (m, 2H), 4,19-4,24 (m, 2H), 4,78-4,82 (m, 1 H), 6,99-7,04 (m, 2H), 7,49 (t, 1 H, J=7,2Hz), 7,60 (d, 1 H, J= 7,6Hz), 8,12 (d, 1 H, J= 2,2Hz), 8,49 (d, 1 H, J= 2Hz).
MS (+ESI) m/z 325 (MH+)

### Exemple 15 (exemple de référence) : 3-[4-(2-Chloro-5-fluoro-phénoxy)-pipéridin-1-yl]-5-phényl-[1,2,4]triazine (15)

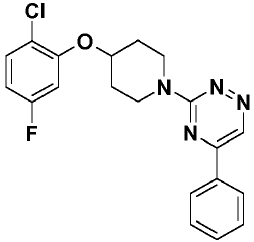

Le composé 15 est préparé à partir du 2-chloro-5-trifluorométhylphénol et de l'intermédiaire 5f selon la méthode de synthèse 3 dans les conditions opératoires décrites pour l'exemple 8 (rendement 28%).
CCM gel de silice 60 F 254 Merck, CH₂Cl₂-MeOH : 95-5, Rf=0,63.
F=61°C
RMN ¹H (CDCl₃) ppm: 1,97-2,07 (m, 4H), 4,18-4,23 (m, 4H), 4,64-4,69 (m, 1H), 6,66 (t, 1 H, J=8Hz), 6,75 (d, 1 H, J=7,2Hz), 7,33 (t, 1 H, J=8,8Hz), 7,50-7,58 (m, 3H), 8,10 (d, 2H, J=8Hz), 9,00 (s, 1 H).
MS (+ESI) m/z 385 (MH+)

### Exemple 16 (exemple de référence) : 5-Phényl-3-[4-(2-trifluorométhyl-phénoxy)-pipéridin-1-yl]-[1,2,4]triazine (16)

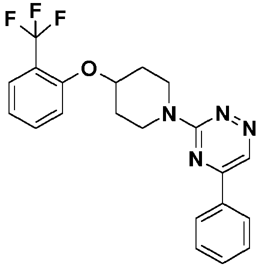

Le composé 16 est préparé à partir du 2-trifluorométhylphénol et de l'intermédiaire 5f selon la méthode de synthèse 3 dans les conditions opératoires décrites pour l'exemple 8 (rendement 24%).
CCM gel de silice 60 F 254 Merck, CH₂Cl₂-MeOH : 95-5, Rf=0,61.
RMN ¹H (CDCl₃) ppm: 2,02-2,06 (m, 4H), 3,99-4,05 (m, 2H), 4,28-4,33 (m, 2H), 4,78-4,83 (m, 1 H), 6,93-7,06 (m, 2H), 7,48-7,61 (m, 5H), 8,10 (d, 2H, J=8Hz), 8,99 (s, 1 H).
MS (+ESI) m/z 401 (MH+)

### Exemple 17(exemple de référence) : 3-[4-(2-Chloro-5-trifluorométhyl-phénoxy)-pipéridin-1-yl]-[1,2,4]triazine (17)

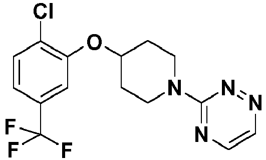

Le composé 17 est préparé à partir du 2-chloro-5-trifluorométhylphénol et de l'intermédiaire 5e selon la méthode de synthèse 3 dans les conditions opératoires décrites pour l'exemple 8 (rendement 55%).
CCM gel de silice 60 F 254 Merck, CH₂Cl₂-MeOH : 95-5, Rf=0,74.
F=66°C
RMN ¹H (CDCl₃) ppm: 1,96-2,04 (m, 4H), 4,02-4,12 (m, 4H), 4,73-4,77 (m, 1H), 7,19-7,21 (m, 2H, J=7,2Hz), 7,51 (d, 1 H, J= 8Hz), 8,13 (d, 1 H, J= 2,4Hz), 8,51 (d, 1 H, J= 2,4Hz).
MS (+ESI) m/z 359 (MH+)

### Exemple 18 (exemple de référence) : 3-[4-(2-Chloro-5-fluoro-phénoxy)-pipéridin-1-yl]-[1,2,4]triazine (18)

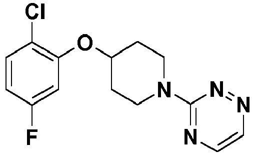

Le composé 18 est préparé à partir du 2-chloro-5-fluorophénol et de l'intermédiaire 5e selon la méthode de synthèse 3 dans les conditions opératoires décrites pour l'exemple 8 (rendement 57%).
CCM gel de silice 60 F 254 Merck, CH₂Cl₂-MeOH : 95-5, Rf=0,57.
RMN ¹H (CDCl₃) ppm: 1,95-2,02 (m, 4H), 4,04-4,11 (m, 4H), 4,63-4,67 (m, 1H), 6,66 (td, 1 H, J=8,4Hz et J=2,4Hz), 6,73 (dd, 1 H, J=10,4Hz et J=2,8Hz), 7,33 (m, 1 H), 8,13 (d, 1 H, J= 2,4Hz), 8,50 (d, 1 H, J= 2Hz).
MS (+APCI) m/z 308 (MH+)

### Exemple 19 (exemple de référence) : 3-[4-(2-Chloro-5-trifluorométhyl-phénoxy)-pipéridin-1-yl]-5,6-diméthyl-[1,2,4]triazine (19)

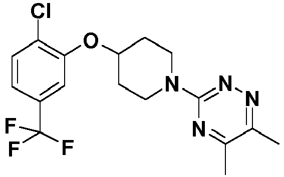

Le composé 19 est préparé à partir du dérivé 3a et de l'intermédiaire 1 b selon la méthode de synthèse 1, sans base, au reflux du THF, dans les conditions opératoires décrites pour l'exemple 1 (rendement: 55%).
CCM gel de silice 60 F 254 Merck, CH₂Cl₂-AcOEt : 50-50, Rf=0,57.
RMN ¹H (DMSO-d₆) ppm: 1,63-1,74 (m, 2H), 1,93-2,04 (m, 2H), 2,35 (s, 3H), 2,42 (s, 3H), 3,68-3,77 (m, 2H), 4,02-4,12 (m, 2H), 4,97-5,05 (m, 1H), 7,34 (d, 1 H, J=8,4Hz), 7,62 (s, 1 H), 7,69 (d, 1 H, J=8,4Hz).
MS (+ESI) m/z 387 (MH+)

### Exemple 20 (exemple de reference) : 3-[4-(2-Chloro-5-trifluorométhyl-phénoxy)-pipéridin-1-yl]-5,6-diphényl-[1,2,4]triazine (20)

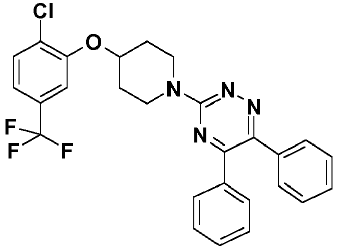

Le composé 20 est préparé à partir du dérivé 3b et de l'intermédiaire 1 b selon la méthode de synthèse 1 dans les conditions opératoires décrites pour l'exemple 19 (rendement : 58%).
CCM gel de silice 60 F 254 Merck, CH₂Cl₂-AcOEt : 50-50, Rf=0,82.
F=75°C
RMN ¹H (DMSO-d₆) ppm: 1,75-1,86 (m, 2H), 2,03-2,15 (m, 2H), 3,88-3,99 (m, 2H), 4,17-4,28 (m, 2H), 5,04-5,12 (m, 1 H), 7,30-7,39 (m, 8H), 7,40-7,47 (m, 3H), 7,66 (s, 1 H), 7,71 (d, 1 H, J=8,4Hz).
MS (+ESI) m/z 511 (MH+)

### Exemple 21 (exemple de référence): 3-[4-(2-Chloro-5-trifluorométhyl-phénoxy)-pipéridin-1-yl]-5,6,7,8-tétrahydro-benzo[1,2,4]triazine (21)

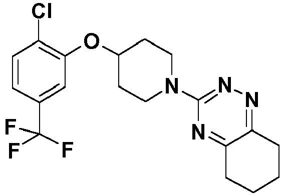

Le composé 21 est préparé à partir du dérivé 3c et de l'intermédiaire 1 b selon la méthode de synthèse 1 dans les conditions opératoires décrites pour l'exemple 19 (rendement : 33%).
CCM gel de silice 60 F 254 Merck, CH₂Cl₂-AcOEt : 50-50, Rf=0,58.
F=153°C
RMN ¹H (DMSO-d₆) ppm: 1,63-1,86 (m, 6H), 1,94-2,03 (m, 2H), 2,71 (t, 2H, J=5,6Hz), 2,85 (t, 2H, J=5,6Hz) 3,62-3,78 (m, 2H), 4,02-4,11 (m, 2H), 4,98-5,05 (m, 1 H), 7,34 (d, 1 H, J=8,4Hz), 7,62 (s, 1 H), 7,69 (d, 1 H, J=8,4Hz).
MS (+ESI) m/z 413 (MH+)

### Exemple 22 (exemple de référence) : 3-[4-(2-Chloro-5-trifluorométhyl-phénoxy)-pipéridin-1-yl]-6-méthyl-5-(3-trifluorométhyl-phényl)-[1,2,4]triazine (22)

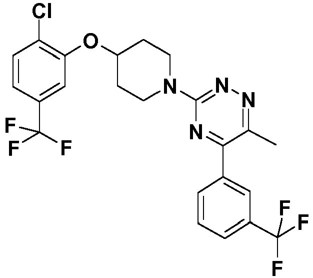

Le composé 22 est préparé à partir du dérivé 3d et de l'intermédiaire 1 b selon la méthode de synthèse 1 dans les conditions opératoires décrites pour l'exemple 19 (rendement : 62%).
CCM gel de silice 60 F 254 Merck, CH₂Cl₂-AcOEt : 50-50, Rf=0,69.
RMN ¹H (DMSO-d₆) ppm: 1,70-1,82 (m, 2H), 2,00-2,10 (m, 2H), 2,42 (s, 3H), 3,82-3,93 (m, 2H), 4,11-4,21 (m, 2H), 5,03-5,10 (m, 1 H), 7,35 (d, 1 H, J=8,2Hz), 7,65 (s, 1 H), 7,69-7,77 (m, 2H), 7,83 (d, 1 H, J=8,0Hz), 7,95 (d, 1 H, J=7,5Hz), 7,98 (s, 1 H).
MS (+ESI) m/z 517 (MH+)

### Exemple 23 (exemple de référence) : 3-[4-(2-Chloro-5-trifluorométhyl-phénoxy)-pipéridin-1-yl]-5,6-di-furan-2-yl-[1,2,4]triazine (23)

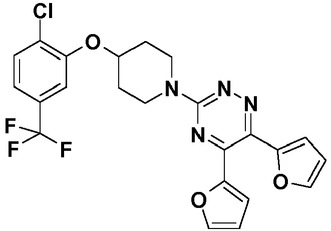

Le composé 23 est préparé à partir du dérivé 3e et de l'intermédiaire 1 b selon la méthode de synthèse 1 dans les conditions opératoires décrites pour l'exemple 19 (rendement : 63%).
CCM gel de silice 60 F 254 Merck, CH₂Cl₂-AcOEt : 50-50, Rf=0,63.
RMN ¹H (DMSO-d₆) ppm: 1,74-1,86 (m, 2H), 2,02-2,13 (m, 2H), 3,89-4,00 (m, 2H), 4,13-4,24 (m, 2H), 5,02-5,11 (m, 1 H), 6,56 (d, 1 H, J=3,4Hz), 6,67-6,72 (m, 2H), 6,81 (d, 1 H, J=3,3Hz), 7,35 (d, 1 H, J=8,4Hz), 7,66 (s, 1 H), 7,72 (d, 1 H, J=8,1 Hz), 7,86 (s, 1 H), 7,97 (s, 1 H).
MS (+ESI) m/z 491 (MH+)

### Exemple 24 (exemple de référence) : 3-[4-(2-Chloro-5-trifluorométhyl-phénoxy)-pipéridin-1-yl]-5,6-di-pyridin-2-yl-[1,2,4]triazine (24)

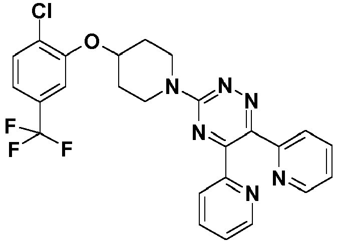

Le composé 24 est préparé à partir du dérivé 3f et de l'intermédiaire 1 b selon la méthode de synthèse 1 dans les conditions opératoires décrites pour l'exemple 19 (rendement : 84%).
CCM gel de silice 60 F 254 Merck, CH₂Cl₂-AcOEt : 50-50, Rf=0,21.
F=130°C
RMN ¹H (DMSO-d₆) ppm: 1,77-1,88 (m, 2H), 2,05-2,15 (m, 2H), 3,93-4,04 (m, 2H), 4,18-4,28 (m, 2H), 5,05-5,13 (m, 1H), 7,26-7,31 (m, 1H), 7,33-7,42 (m, 2H), 7,67 (s, 1 H), 7,71 (d, 1 H, J=8,4Hz), 7,86-7,96 (m, 4H), 8,24 (d, 1 H, J=4,8Hz), 8,30 (d, 1 H, J=4,8Hz).
MS (+ESI) m/z 513 (MH+)

### Exemple 25 (exemple de référence) : 3-[4-(2-Chloro-5-fluoro-phénoxy)-pipéridin-1-yl]-5-méthyl-[1,2,4]triazine (25)

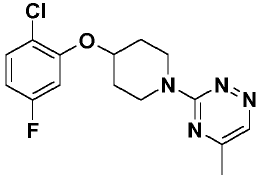

Le composé 25 est préparé à partir de la triazine 2c et de l'intermédiaire 1c selon la méthode de synthèse 1 dans les conditions opératoires décrites pour l'exemple 1 (rendement 12%).
CCM gel de silice 60 F 254 Merck, CH₂Cl₂-AcOEt : 95-5, Rf=0,28.
F=92°C
RMN ¹H (CDCl₃) ppm: 1,92-2,05 (m, 4H), 2,36 (s, 3H), 4,02-4,10 (m, 4H), 4,62-4,65 (m, 1 H), 6,63-6,69 (m, 1H), 6,70-6,75 (m, 1H), 7,30-7,35 (m, 1H), 8,40 (s, 1 H).
MS (+ESI) m/z 323 (MH+)

### Exemple 26 (exemple de référence) : 5-Méthyl-3-(4-o-tolyloxy-pipéridin-1-yl)-[1,2,4]triazine (26)

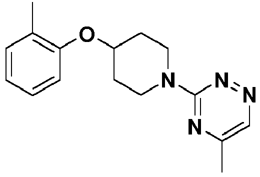

Le composé 26 est préparé à partir de la triazine 2c et de l'intermédiaire 1g selon la méthode de synthèse 1 dans les conditions opératoires décrites pour l'exemple 1 (rendement 22%).
CCM gel de silice 60 F 254 Merck, CH₂Cl₂-AcOEt : 95-5, Rf=0,27.
F=100°C
RMN ¹H (CDCl₃) ppm: 1,87-1,95 (m, 2H), 1,98-2,05 (m, 2H), 2,25 (s, 3H), 2,35 (s, 3H), 3,94-4,00 (m, 2H), 4,07-4,13 (m, 2H), 4,62-4,65 (m, 1 H), 6,87 (m, 2H), 7,16 (m, 2H), 8,39 (s, 1 H).
MS (+ESI) m/z 285 (MH+)

### Exemple 27 (exemple de reference) : 3-[4-(2-Chloro-5-trifluorométhyl-phénoxy)-pipéridin-1-yl]-5-méthyl-[1,2,4]triazine (27)

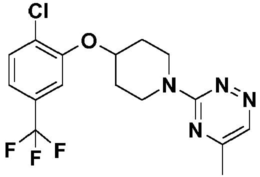

Le composé 27 est préparé à partir de la triazine 2c et de l'intermédiaire 1b selon la méthode de synthèse 1 dans les conditions opératoires décrites pour l'exemple 1 (rendement 11%).
CCM gel de silice 60 F 254 Merck, CH₂Cl₂-AcOEt : 95-5, Rf=0,3.
F=68°C
RMN ¹H (CDCl₃) ppm: 1,92-2,04 (m, 4H), 2,36 (s, 3H), 4,04-4,12 (m, 4H), 4,71-4,75 (m, 1 H), 7,18-7,20 (m, 2H), 7,16 (d, 1 H, J=8,8Hz), 8,41 (s, 1 H).
MS (+ESI) m/z 373 (MH+)

### Exemple 28 (exemple de référence) : 3-[4-(2-Chloro-phénoxy)-pipéridin-1-yl]-5-méthyl-[1,2,4]triazine (28)

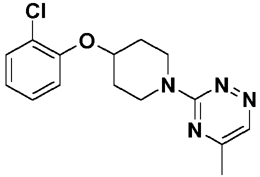

Le composé 28 est préparé à partir de la triazine 2c et de l'intermédiaire 1d selon la méthode de synthèse 1 dans les conditions opératoires décrites pour l'exemple 1 (rendement 10%).
CCM gel de silice 60 F 254 Merck, CH₂Cl₂-AcOEt : 95-5, Rf=0,23.
F=118°C
RMN ¹H (CDCl₃) ppm: 1,92-2,04 (m, 4H), 2,35 (s, 3H), 3,98-4,04 (m, 2H), 4,09-4,15 (m, 2H), 4,64-4,67 (m, 1 H), 6,93 (t, 1 H, J=7,6Hz), 7,00 (d, 1 H, J=8Hz), 7,22 (t, 1 H, J=7,6Hz), 7,39 (d, 1 H, J=8Hz), 8,39 (s, 1 H).
MS (+ESI) m/z 305 (MH+)

### Exemple 29 (exemple de référence): 3-(4-o-Tolyloxy-pipéridin-1-yl)-[1,2,4]triazine (29)

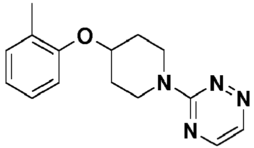

Le composé 29 est préparé à partir de la triazine 2a et de l'intermédiaire 1g selon la méthode de synthèse 1 dans les conditions opératoires décrites pour l'exemple 1 (rendement 36%).
CCM gel de silice 60 F 254 Merck, CH₂Cl₂-AcOEt : 95-5, Rf=0,22.
RMN ¹H (CDCl₃) ppm: 1,90-2,06 (m, 4H), 2,26 (s, 3H), 3,98-4,12 (m, 4H), 4,65-4,68 (m, 1 H), 6,86-6,90 (m, 2H), 7,16 (m, 2H), 8,12 (d, 1 H, J=2Hz), 8,49 (d, 1 H, J=2,4Hz).
MS (+ESI) m/z 271 (MH+)

### Exemple 30 (exemple de référence) : 3-[4-(2-Chloro-5-fluoro-phénoxy)-pipéridin-1-yl]-6-méthyl-[1,2,4]triazine (30)

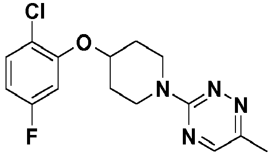

Le composé 30 est préparé à partir de la triazine 2j et de l'intermédiaire 1c selon la méthode de synthèse 1 dans les conditions opératoires décrites pour l'exemple 1 (rendement 8%).
CCM gel de silice 60 F 254 Merck, CH₂Cl₂-AcOEt : 90-10, Rf=0,28.
RMN ¹H (CDCl₃) ppm: 1,91-2,05 (m, 4H), 2,51 (s, 3H), 3,96-4,10 (m, 4H), 4,60-4,63 (m, 1 H), 6,65 (td, 1H, J=8,8Hz et J=2,8Hz), 6,72 (dd, 1H, J=10Hz et J=2,8Hz), 7,30-7,34 (m, 1 H), 8,03 (s, 1 H).
MS (+ESI) m/z 323 (MH+)

### Exemple 31 (exemple de référence) : 6-Méthyl-3-(4-o-tolyloxy-pipéridin-1-yl)-[1,2,4]triazine (31)

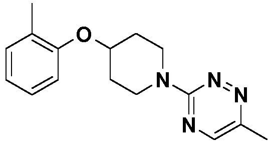

Le composé 31 est préparé à partir de la triazine 2j et de l'intermédiaire 1g selon la méthode de synthèse 1 dans les conditions opératoires décrites pour l'exemple 1 (rendement 6%).
CCM gel de silice 60 F 254 Merck, CH₂Cl₂-AcOEt : 90-10, Rf=0,27.
RMN ¹H (CDCl₃) ppm: 1,87-1,93 (m, 2H), 1,98-2,04 (m, 2H), 2,25 (s, 3H), 2,51 (s, 3H), 3,89-3,95 (m, 2H), 4,04-4,10 (m, 2H), 4,60-4,64 (m, 1H), 6,87 (m, 2H), 7,15 (m, 2H), 8,02 (s, 1 H).
MS (+ESI) m/z 285 (MH+)

### Exemple 32 (exemple de reference) : 3-[4-(2-Chloro-5-nitro-phénoxy)-pipéridin-1-yl]-[1,2,4]triazine (32)

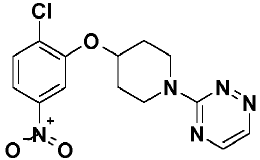

Le composé 32 est préparé à partir du 2-chloro-5-nitrophénol et de l'intermédiaire 5e selon la méthode de synthèse 3 dans les conditions opératoires décrites pour l'exemple 8 (rendement 54%).
CCM gel de silice 60 F 254 Merck, CH₂Cl₂-AcOEt : 95-5, Rf=0,26.
RMN ¹H (CDCl₃) ppm: 1,97-2,12 (m, 4H), 4,04-4,16 (m, 4H), 4,82-4,85 (m, 1H), 7,56 (d, 1 H, J=8,4Hz), 7,83 (d, 1 H, J= 7,6Hz), 7,84 (s, 1 H), 8,14 (d, 1 H, J= 2Hz), 8,52 (d, 1 H, J= 2Hz).
MS (+ESI) m/z 336 (MH+)

### Exemple 33 (exemple de référence) : 3-[4-(2-Bromo-4,5-difluoro-phénoxy)-pipéridin-1-yl]-[1,2,4]triazine (33)

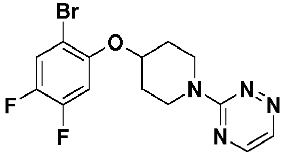

Le composé 33 est préparé à partir du 2-bromo-4,5-difluoro-phénol et de l'intermédiaire 5e selon la méthode de synthèse 3 dans les conditions opératoires décrites pour l'exemple 8 (rendement 41%).
CCM gel de silice 60 F 254 Merck, CH₂Cl₂-AcOEt : 95-5, Rf=0,28.
RMN ¹H (CDCl₃) ppm: 1,93-2,03 (m, 4H), 4,01-4,14 (m, 4H), 4,56-4,62 (m, 1H), 6,80-6,85 (m, 1 H), 7,39-7,44 (m, 1 H), 8,13 (d, 1 H, J= 2Hz), 8,51 (d, 1 H, J= 2Hz).
MS (+ESI) m/z 370 (MH+)

### Exemple 34 (exemple de référence) : 3-[4-(3-Fluoro-5-trifluorométhyl-phénoxy)-pipéridin-1-yl]-[1,2,4]triazine (34)

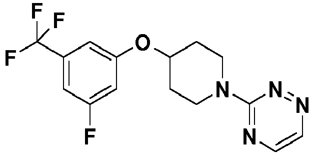

Le composé 34 est préparé à partir du 3-fluoro-5- trifluorométhyl-phénol et de l'intermédiaire 5e selon la méthode de synthèse 3 dans les conditions opératoires décrites pour l'exemple 8 (rendement 9%).
CCM gel de silice 60 F 254 Merck, CH₂Cl₂-AcOEt : 95-5, Rf=0,32.
RMN ¹H (CDCl₃) ppm: 1,86-1,93 (m, 2H), 2,03-2,09 (m, 2H), 3,93-3,98 (m, 2H), 4,13-4,18 (m, 2H), 4,63-4,66 (m, 1 H), 6,82 (d, 1 H, J=10,4Hz), 6,94 (d, 1 H, J= 8,4Hz), 6,98 (s, 1 H), 8,14 (d, 1 H, J= 2Hz), 8,52 (d, 1 H, J= 2Hz).
MS (+ESI) m/z 343 (MH+)

### Exemple 35 (exemple de référence) : 3-[4-(2-Nitro-phénoxy)-pipéridin-1-yl]-[1,2,4]triazine (35)

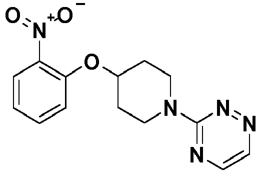

Le composé 35 est préparé à partir du 2-nitro-phénol et de l'intermédiaire 5e selon la méthode de synthèse 3 dans les conditions opératoires décrites pour l'exemple 8 (rendement 36%).
CCM gel de silice 60 F 254 Merck, CH₂Cl₂-AcOEt : 95-5, Rf=0,19.
RMN ¹H (CDCl₃) ppm: 1,99-2,04 (m, 4H), 3,95-4,01 (m, 2H), 4,15-4,20 (m, 2H), 4,80-4,85 (m, 1H), 7,05 (t, 1H, J=7,6Hz), 7,13 (d, 1H, J= 8,4Hz), 7,53 (t, 1H, J=8,4Hz), 7,84 (d, 1 H, J=8Hz), 8,12 (d, 1 H, J= 2Hz), 8,50 (d, 1 H, J= 2Hz).
MS (+ESI) m/z 302 (MH+)

### Exemple 36 (exemple de référence): 1-[1,2,4]Triazin-3-yl-pipéridin-4-yl)-(2-trifluorométhyl-phényl)-amine (36)

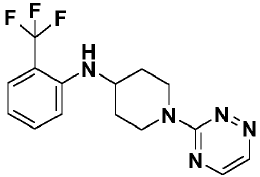

Le composé 36 est préparé selon la méthode de synthèse 3: 0,5g (2,78mmoles) d'intermédiaire 5b sont placés dans 10mL de toluène en présence de 0,31g (3,25mmoles) de tert-butoxyde de sodium, de 0,011 g (1,16mmoles) de dipalladium tris(dibenzylidène-acétone), de 0,021g (3,45mmoles) de bis-diphénylphospino-1,1'-binaphtyl et de 0,52g (2,32mmoles) de 2-trifluorométhyl-bromo-benzène. Le milieu est chauffé à 110°C pendant 40h. Après filtration sur célite du milieu, le filtrat est concentré à sec. Le résidu obtenu est purifié par chromatographie flash sur silice (gradient CH₂Cl₂-AcOEt : 100-0 à 95-5 sur 30 min). 0,19g de solide rosé sont obtenus (rendement 25%).
CCM gel de silice 60 F 254 Merck, CH₂Cl₂-AcOEt : 95-5, Rf=0,29.
F=80°C
RMN ¹H (CDCl₃) ppm: 1,49-1,59 (m, 2H), 2,17-2,21 (m, 2H), 3,31-3,38 (m, 2H), 3,69-3,76 (m, 1 H), 4,23-4,26 (m, 1 H), 4,67-4,71 (m, 2H), 6,73 (t, 1 H, J=7,6Hz), 6,82 (d, 1 H, J= 8,4Hz), 7,38 (t, 1 H, J=8Hz), 7,45 (d, 1 H, J= 7,6Hz), 8,13 (d, 1 H, J= 2,4Hz), 8,51 (d, 1 H, J= 2,4Hz).
MS (+ESI) m/z 324 (MH+)

### Exemple 37 (exemple de référence) : 3-[4-(2-Chloro-5-trifluorométhyl-phénoxy)-3-méthyl-pipéridin-1-yl]-[1,2,4]triazine (37)

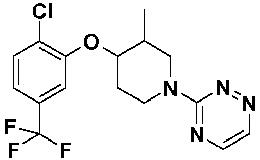

Le composé 37 est préparé à partir du 2-chloro-5-trifluorométhylphénol et de l'intermédiaire 5c selon la méthode de synthèse 3 dans les conditions opératoires décrites pour l'exemple 8 (rendement 38%).
CCM gel de silice 60 F 254 Merck, CH₂Cl₂-MeOH : 95-5, Rf=0,84.
RMN ¹H (CDCl₃) ppm: 1,12 (d, 3H, J=6,8Hz), 2,07-2,17 (m, 2H), 3,46-3,53 (m, 2H), 4,55-4,65 (m, 3H), 7,17-7,19 (m, 2H), 7,49-7,52 (m, 1H), 8,12-8,14 (m, 1 H), 8,49-8,51 (m, 1 H).
MS (+ESI) m/z 373 (MH+)

### Exemple 38 (exemple de référence) : 3-[4-(2,5-Dichloro-phénoxy)-3-méthyl-pipéridin-1-yl]-[1,2,4]triazine (38)

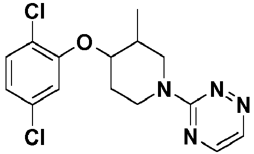

Le composé 38 est préparé à partir du 2,5-dichlorophénol et de l'intermédiaire 5c selon la méthode de synthèse 3 dans les conditions opératoires décrites pour l'exemple 8 (rendement 37%).
CCM gel de silice 60 F 254 Merck, CH₂Cl₂-MeOH : 95-5, Rf=0,85.
RMN ¹H (CDCl₃) ppm: 1,12 (d, 3H, J=7,2Hz), 2,06-2,18 (m, 2H), 3,46-3,53 (m, 2H), 4,53-4,57 (m, 3H), 6,87-6,91 (m, 1 H), 6,95-6,96 (m, 1 H), 7,29-7,32 (m, 1 H), 8,11-8,13 (m, 1 H), 8,48-8,50 (m, 1 H).
MS (+ESI) m/z 339 (MH+)

### Exemple 39 (exemple de référence) : 5-[4-(2-Chloro-5-trifluorométhyl-phénoxy)-pipéridin-1-yl]-2-méthyl-2H-pyridazin-3-one (39)

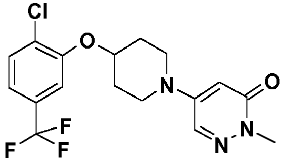

Le composé 39 est préparé à partir du dérivé 4a et de l'intermédiaire 1 b selon la méthode de synthèse 1, en utilisant comme solvant de l'éthanol sous champ de micro-ondes à 120°C pendant 20min, dans les conditions opératoires décrites pour l'exemple 1 (rendement : 19%).
CCM gel de silice 60 F 254 Merck, AcOEt, Rf=0,24.
F=130°C
RMN ¹H (DMSO-d₆) ppm: 1,64-1,75 (m, 2H), 1,93-2,03 (m, 2H), 3,32-3,41 (m, 2H), 3,51 (s, 3H), 3,55-3,65 (m, 2H), 3,92-5,00 (m, 1 H), 5,92 (d, 1 H, J=2,8Hz), 7,33 (dd, 1 H, J=8,0Hz, J=1,2Hz), 7,61 (d, 1 H, J=1,2Hz), 7,69 (d, 1 H, J=8,4Hz), 7,97 (d, 1 H, J=2,8Hz).
MS (+ESI) m/z 388 (MH+)

### Exemple 40 (exemple de référence) : 4-[4-(2-Chloro-5-trifluorométhyl-phénoxy)-pipéridin-1-yl]-2-méthyl-2H-pyridazin-3-one (40)

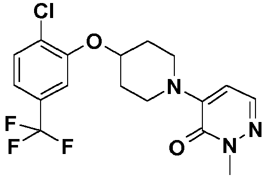

Le composé 40 est préparé à partir du dérivé 4b et de l'intermédiaire 1 b selon la méthode de synthèse 1, en utilisant comme solvant l'acétonitrile au reflux, dans les conditions opératoires décrites pour l'exemple 1 (rendement : 87%). CCM gel de silice 60 F 254 Merck, Ether de pétrole-AcOEt: 60-40, Rf=0,35.
F=121°C
RMN ¹H (DMSO-d₆) ppm: 1,69-1,81 (m, 2H), 1,96-2,07 (m, 2H), 3,36-3,47 (m, 2H), 3,61 (s, 3H), 3,66-3,76 (m, 2H), 4,91-,4,99 (m, 1 H), 6,55 (d, 1 H, J=4,9Hz), 7,33 (d, 1 H, J=8,6Hz), 7,61 (s, 1 H), 7,64 (d, 1 H, J=4,8Hz), 7,69 (d, 1 H, J=8,0Hz).
MS (+ESI) m/z 388 (MH+)

### Exemple 41 (exemple de référence) : 3-[3-(2-Chloro-5-trifluorométhyl-phénoxyméthyl)-azetidin-1-yl]-[1,2,4]triazine (41)

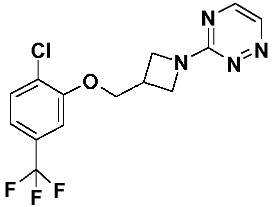

Le composé 41 est préparé à partir de la triazine 2a et de l'intermédiaire 1e selon la méthode de synthèse 1 dans les conditions opératoires décrites pour l'exemple 1 (rendement 55%).
CCM gel de silice 60 F 254 Merck, CH₂Cl₂-AcOEt : 80-20, Rf=0,5.
RMN ¹H (DMSO-d₆) ppm: 3,24-3,28 (m, 1 H), 4,07-4,10 (m, 2H), 4,28-4,32 (m, 2H), 4,43 (d, 2H, J=6Hz), 7,32-7,36 (m, 1 H), 7,51 (d, 1 H, J=3Hz), 7,66 (dd, 1 H, J=3Hz et J=9Hz), 8,34 (d, 1 H, J=3Hz), 8,64 (d, 1 H, J=3Hz).

### Exemple 42 (exemple de référence) : 3-[3-(2-Chloro-5-trifluorométhyl-phénoxy)-pyrrolidin-1-yl]-[1,2,4]triazine (42)

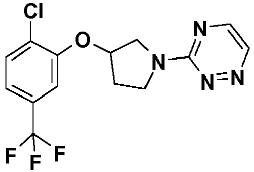

Le composé 42 est préparé à partir du 2-chloro-5-trifluorométhylphénol et de l'intermédiaire 5d selon la méthode de synthèse 3 dans le toluène en présence de DIAD, dans les conditions opératoires décrites pour l'exemple 8 (rendement 86%).
CCM gel de silice 60 F 254 Merck, Cyclohexane-AcOEt : 60-40, Rf=0,4.
RMN ¹H (DMSO-d₆) ppm: 2,21-2,42 (m, 2H), 3,60-3,90 (m, 4H), 5,48-5,49 (m, 1 H), 7,33-7,37 (m, 1H), 7,63-7,68 (m, 2H), 8,33 (d, 1H, J=3Hz), 8,63 (d, 1H, J=3Hz).

### Exemple 43 (exemple de référence) : [(trans)-2-(2-Chloro-5-trifluorométhyl-phénoxyméthyl)-cyclopropyl]-[1,2,4]triazin-3-yl-amine (43)

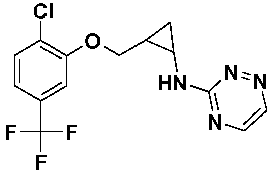

Le composé 43 est préparé à partir de la triazine 3g et de l'intermédiaire 1f selon la méthode de synthèse 1 dans les conditions opératoires décrites pour l'exemple 1 (rendement 8%).
CCM gel de silice 60 F 254 Merck, CH₂Cl₂-AcOEt : 85-15, Rf=0,4.
RMN ¹H (DMSO-d₆) ppm: 0,90-0,92 (m, 2H), 1,47-1,5 (m, 1 H), 2,86-2,91 (m, 1 H), 4,07-4,11 (m, 1H), 4,30-4,34 (m, 1H), 7,32 (d, 1H, J=9Hz), 7,46 (d, 1H, J=3Hz), 7,68 (d, 1 H, J=9Hz), 8,00-8,02 (s large, 1 H), 8,27 (d, 1 H, J=3Hz), 8,63 (d, 1 H, J=3Hz).
MS (+APCI) m/z 345 (MH+)

### EVALUATION PHARMACOLOGIQUE

Activité enzymatique humaine de SCD-1 à partir de microsomes de cellules HepG2 après traitement par les composés inhibiteurs (% d'inhibition) :
Les cellules HepG2 d'hépatocarcinome humain (ATCC, HB-8065) sont cultivées à confluence puis trypsinisées. Le culot cellulaire est repris dans du tampon Tris 10 mM (pH 7,4) sucrose (250 mM) DTT (1 mM) puis les cellules sont lysées par sonication. Les microsomes sont obtenus après une centrifugation à 10.000 g pendant 20 minutes à 4°C suivie d'une centrifugation du surnageant à 100.000 g pendant 60 minutes à 4°C. Le culot est repris dans du tampon Tris 10 mM (pH 7,4) sucrose (250 mM) à 4°C et les protéines microsomales sont dosées et stockées à -196°C (azote liquide).

La réaction enzymatique mesure la conversion de l'acide stéarique (acide gras en C18:0) en acide oléique (acide gras en C18:1) par la SCD-1. La réaction enzymatique est démarrée par l'addition de 125 µg de fraction microsomale de cellules HepG2 à des tubes (volume réactionnel total de 500 µl) contenant 62 µM d'acide stéarique (45 µM d'acide stéarique et 17 µM d'[¹⁴C] acide stéarique) dans un tampon phosphate à 100 mM (pH 7,16) avec 7,2 mM d'ATP, 0,54 mM de CoA, 6 mM de MgCl₂, 0,8 mM de NADH et le composé inhibiteur ou le véhicule (0.1% DMSO). Les tubes sont incubés pendant 20 minutes à 37°C puis la réaction enzymatique est arrêtée par addition de KOH (12%) et saponification de 30 minutes à 80°C. Après acidification (HCl 3N), les acides gras sont extraits deux fois par de l'éther éthylique, évaporés sous azote avant d'être repris par du méthanol/dichlorométhane (3:1). Le produit de la réaction (C18:1) est séparé du substrat de la réaction (C18:0) par HPLC (Perkin Elmer, colonne en phase inverse C18) couplé à un détecteur de radioactivité en ligne (FlowOne). L'activité enzymatique est calculée en picomoles d'acide stéarique transformées en acide oléique par minute et par mg de protéine. Pour chaque composé inhibiteur, une IC₅₀ est déterminée par rapport à l'activité enzymatique de référence (véhicule 0.1% DMSO). L'acide sterculique est le composé inhibiteur de référence (Gomez F.E., Bauman D.E., Ntambi J.M., Fox B.G. Effects of sterculic acid on stearoyl-CoA desaturase in differentiating 3T3-L1 adipocytes. Biochem Biophys Res Commun. 300 316-326 (2003).

**Tableau 5 : Activité enzymatique humaine de SCD-1.**

| Exemples | HSCD-1 (HEPG2) IC₅₀ µM |
|---|---|
| Acide sterculique | 0,3 |
| 6 | 0,1-1 |
| 7 | 0,1-0,3 |
| 8 | 0,1-0,3 |
| 9 | 0,1-1 |
| 11 | 0,1 |
| 12 | 0,01-0,03 |
| 13 | 0,1-1 |
| 14 | 0,1-1 |
| 17 | 0,1 |
| 18 | 0,03 |
| 19 | 0,1-1 |
| 25 | 0,1 |
| 27 | 0,3 |
| 28 | 0,3-1 |
| 30 | 0,1 |
| 32 | 0,03-0,1 |
| 33 | 0,01-0,1 |
| 35 | 0,1-1 |
| 36 | 0,03 |
| 37 | 0,1-1 |
| 38 | 0,3-1 |
| 40 | 0,1-0,3 |
| 42 | 0,3-1 |

Les résultats obtenus montrent que les composés de formule générale (I) inhibent l'activité enzymatique de l'enzyme SCD-1.

Les composés de formule générale (I) peuvent être utilisés en tant qu'inhibiteurs de l'enzyme SCD-1.

Application topique de composés inhibiteurs de SCD-1 chez la souris NMRI : réduction du nombre et de la taille des glandes sébacées.

Protocole expérimental : des souris mâles Crl:NMRI (24-26 g) sont rasées (2 x 2 cm² minimum) 2-3 jours avant la première application des molécules à tester. Pour prévenir l'absorption orale potentielle des molécules, les animaux sont hébergés individuellement.

Les molécules sont solubilisées dans le véhicule éthanol/propylène glycol (30/70, v/v) à une concentration maximale de 1%. 50 µL de chaque préparation (molécule à tester, ou véhicule) sont appliqués 2 fois par jour pendant 5 jours consécutifs sur la zone rasée de 2 cm², avec le cône d'une pipette par plusieurs allers-retours. Au minimum 6 heures après la dernière application, les souris sont euthanasiées, les prélèvements de peau effectués et fixés immédiatement afin de procéder à la réalisation de coupes en paraffine ; une analyse histomorphométrique après coloration par Hematoxyline/Eosine est réalisée.

Pour chaque animal au moins 3 sections d'environ 10 mm de longueur sont lues en aveugle par 3 personnes, et l'efficacité des molécules sur l'atrophie des glandes sébacées est évaluée quantitativement (nombre) et qualitativement (score relatif, taille). La durée d'action, la réversibilité de l'effet sont vérifiées de la même façon en modifiant les séquences d'application.

**Tableau 6 : application topique de composés inhibiteurs de SCD-1 (souris NMRI): réduction du nombre et de la taille des glandes sébacées.**

| Exemples | Concentration (%) | Temps de traitement | ↘ nombre de glandes sébacées (%) |
|---|---|---|---|
| 6 | 0,3 | 5 jours | 79 |
| 7 | 0,3 | 5 jours | 75 |
| 8 | 0,3 | 5 jours | 95 |
| 9 | 0,3 | 5 jours | 43 |
| 11 | 1 | 5 jours | 45 |
| 12 | 0,3 | 5 jours | 94 |
| 17 | 1 | 5 jours | 97 |
| 40 | 0,1 | 5 jours | 36 |

Activités cytotoxiques vis-à-vis de lignées cellulaires humaine de cancer du colon (HCT-116) :
Les cellules tumorales HCT-116 sont ensemencées en plaque 96 puits dans du milieu RPMI 1640 auquel est ajouté 5% de sérum de veau foetal (100µl/puits, respectivement à 1.5 10⁴ cellules/ml). Après une incubation de 24h à 37°C dans un incubateur à 5% CO₂, 11µL de milieu contenant le composé à tester à une concentration 10 fois supérieure à la concentration finale sont ajoutés. Les plaques sont incubées 72h supplémentaires. La survie cellulaire est évaluée par mesure de la luminescence après relargage de l'ATP dans le milieu en utilisant les solutions de lyse cellulaire, de luciférase et de luciférine contenus dans le kit ATP-lite-M™ comme cela est recommandé par le fabricant (Packard, Rungis, France). Chaque condition expérimentale a été répliquée au moins trois fois indépendamment avec une lecture des puits en sextuplet par pas de dose. Les résultats montrent que les composés de l'invention présentent de puissantes propriétés cytotoxiques. La concentration inhibitrice 50 (EC₅₀), concentration du composé inhibant la prolifération cellulaire de 50%, est de 60nM pour le composé 12 et de 230nM pour le composé 17 sur les cellules de lignées tumorales HCT-116.

**Tableau 7 : Activité anti-proliférative de composés inhibiteurs de SCD-1, vis-à vis de la lignée tumorale humaine HTC116 :**

| Exemples | EC₅₀ (µM) |
|---|---|
| 12 | 0,06 |
| 17 | 0,23 |

## Revendications

1. Composé de formule générale I 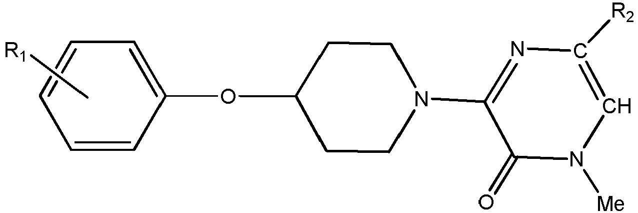 dans laquelle :
- R₁ représente un ou plusieurs substituant(s) choisi(s) parmi trifluorométhtyle, F, Cl, Br, méthyle et nitro,
- R₂ représente Cl ou H
ainsi que leurs sels pharmaceutiquement acceptables.

2. Dérivés de formule générale I selon la revendication 1, **caractérisés en ce qu'**ils sont choisis parmi :
1. 5-Chloro-3-[4-(2-chloro-phénoxy)-pipéridin-1-yl]-1-méthyl-1H-pyrazin-2-one
2. 5-Chloro-1-méthyl-3-[4-(2-trifluorométhyl-phénoxy)-pipéridin-1-yl]-1H-pyrazin-2-one
3. 5-Chloro-3-[4-(2-chloro-5-trifluorométhyl-phénoxy)-pipéridin-1-yl]-1-méthyl-1H-pyrazin-2-one
4. 5-Chloro-1-méthyl-3-(4-o-tolyloxy-pipéridin-1-yl)-1H-pyrazin-2-one
5. 1-Méthyl-3-(4-o-tolyloxy-pipéridin-1-yl)-1H-pyrazin-2-one
6. 1-Méthyl-3-[4-(2-trifluorométhyl-phénoxy)-pipéridin-1-yl]-1H-pyrazin-2-one
7. 5-Chloro-3-[4-(2-chloro-5-fluoro-phénoxy)-pipéridin-1-yl]-1-méthyl-1H-pyrazin-2-one.

3. Composés de formule générale I tels que définis selon l'une des revendications 1) et 2) pour leur utilisation en tant que médicament.

4. Composés de formule générale I tels que définis selon l'une des revendications 1) et 2) pour leur utilisation en tant que principe actif cosmétique.

5. Composés de formule générale I selon la revendication 3), pour leur utilisation en tant qu'inhibiteur de l'enzyme SCD-1.

6. Composés de formule générale I selon la revendication 3), pour leur utilisation en tant que médicament destiné au traitement et/ou la prévention des maladies nécessitant des inhibiteurs de l'activité de l'enzyme SCD-1.

7. Composés de formule générale I selon la revendication 3), pour leur utilisation en tant que médicament destiné au traitement et/ou à la prévention de maladies telles que l'obésité, le diabète de type 2, les dyslipidémies diabétiques, l'hypertriglycéridémie, l'hypercholestérolémie, le syndrome métabolique, l'athérosclérose, la stéatose hépatique ou les risques cardiovasculaires.

8. Composés de formule générale I selon la revendication 3), pour leur utilisation en tant que médicament destiné au traitement et/ou à la prévention de états pathologiques liés à des désordres lipidiques de la peau et aux complications inflammatoires et microbiennes.

9. Composés de formule générale I selon la revendication 3), pour leur utilisation en tant que médicament destiné au traitement et/ou à la prévention des dérèglements de la production et/ou de la sécrétion de sébum associés à une hyperandrogénie.

10. Composés de formule générale I selon la revendication 3), pour leur utilisation en tant que médicament destiné au traitement et/ou à la prévention l'acné, le psoriasis, l'hirsutisme, la rosacée, la dermite séborrhéique, I' hyperséborrhée ou l'eczéma.

11. Composés de formule générale I selon la revendication 3), pour leur utilisation en tant que médicament destiné au traitement et/ou à la prévention du cancer.

12. Composés de formule générale I selon la revendication 3), pour leur utilisation en tant que médicament destiné au traitement et/ou à la prévention des tumeurs liquides et/ou des tumeurs solides, tels que les mélanomes, les cancers colorectaux, les cancers du poumon, de la prostate, de la vessie, du sein, de l'utérus, de l'oesophage, de l'estomac, du pancréas, du foie, les cancers ovariens, les leucémies en particulier les lymphomes et les myélomes, les cancers de la sphère ORL et les cancers du cerveau.

13. Composition pharmaceutique **caractérisée en ce qu'**elle contient à titre de principe actif un composé de formule générale I tel que défini selon l'une des revendications 1) à 2).

14. Composition pharmaceutique **caractérisée en ce qu'**elle contient un composé défini de formule générale I tel que défini selon la revendication 13) en association avec tout excipient approprié.

15. Composition pharmaceutique selon l'une des revendications 13 ou 14, en association avec un anti-diabétique tel que les biguanides, les diverses formes d'insuline, les sulfonylurées, les meglitinides, les modulateurs de PPAR, les inhibiteurs de l'alpha-glucosidase, les inhibiteurs de DPP-4, les analogues d'amyline, les analogues de glucagon-like peptide-1, les inhibiteurs de SGLT2 ou les inhibiteurs de 11β-HSD1.

16. Composition pharmaceutique selon l'une des revendications 13 ou 14, en association avec un anti-obésité tel que l'orlistat ou la sibutramine.

17. Composition pharmaceutique selon l'une des revendications 13 ou 14, en association avec un composé utile dans le traitement ou la prévention des états pathologiques liés à des désordres lipidiques de la peau et aux complications inflammatoires et microbiennes ou des dérèglements de la production et/ou de la sécrétion de sébum associés à une hyperandrogénie tel que les rétinoïdes, les antibiotiques, les antibactériens, ou les anti-androgènes.

18. Composition pharmaceutique selon l'une des revendications 13 ou 14, en association avec d'autres médicaments anticancéreux, qu'ils soient cytotoxiques et/ou cytostatiques, tels que les dérivés du platine, les taxanes, les vincas, le 5-FU, pour augmenter l'efficacité thérapeutique en vue du traitement des tumeurs réfractaires aux thérapeutiques usuelles.

19. Composition pharmaceutique selon l'une des revendications 13 ou 14, **caractérisée en ce qu'**elle est présentée sous une forme apte à l'administration par voie topique.

## Patentansprüche

1. Verbindung der allgemeinen Formel I 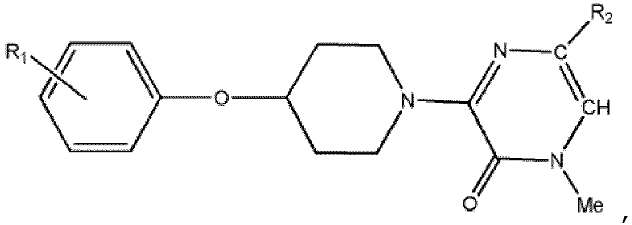 wobei:
- R₁ für einen oder mehrere Substituenten steht, der bzw. die aus Trifluormethyl, F, Cl, Br, Methyl und Nitro ausgewählt ist bzw. sind,
- R₂ für Cl oder H steht,
sowie ihre pharmazeutisch akzeptablen Salze.

2. Derivate der allgemeinen Formel I nach Anspruch 1, **dadurch gekennzeichnet, dass** sie aus folgenden ausgewählt sind:guindeuil@regimbeau.eu
1) 5-Chlor-3-[4-(2-chlorphenoxy)piperidin-1-yl]-1-methyl-1H-pyrazin-2-on
2) 5-Chlor-1-methyl-3-[4-(2-trifluormethylphenoxy)piperidin-1-yl]-1H-pyrazin-2-on
3) 5-Chlor-3-[4-(2-chlor-5-trifluormethylphenoxy)piperidin-1-yl]-1-methyl-1H-pyrazin-2-on
4) 5-Chlor-1-methyl-3-(4-o-tolyloxypiperidin-1-yl)-1H-pyrazin-2-on
5) 1-Methyl-3-(4-o-tolyloxypiperidin-1-yl)-1H-pyrazin-2-on
6) 1-Methyl-3-(4-(2-trifluormethylphenoxy)piperidin-1-yl)-1H-pyrazin-2-on
7) 5-Chlor-3-[4-(2-chlor-5-fluorphenoxy)piperidin-1-yl]-1-methyl-1H-pyrazin-2-on.

3. Verbindungen der allgemeinen Formel I, wie die in einem der Ansprüche 1 und 2 definierten, zu deren Verwendung als Arzneimittel.

4. Verbindungen der allgemeinen Formel I, wie in einem der Ansprüche 1 und 2 definiert, zu deren Verwendung als kosmetischer Wirkstoff.

5. Verbindungen der allgemeinen Formel I nach Anspruch 3 zu deren Verwendung als Hemmer des Enzyms SCD-1.

6. Verbindungen der allgemeinen Formel I nach Anspruch 3 zu deren Verwendung als Arzneimittel, das zur Behandlung und/oder Verhinderung von Krankheiten vorgesehen ist, die Hemmer der Aktivität des Enzyms SCD-1 erfordern.

7. Verbindungen der allgemeinen Formel I nach Anspruch 3 zu deren Verwendung als Arzneimittel, das zur Behandlung und/oder Verhinderung von Krankheiten vorgesehen ist, wie Adipositas, Typ-2-Diabetes, diabetische Dyslipidämien, Hypertriglyzeridämie, Hypercholesterinämie, metabolisches Syndrom, Atherosklerose, Leberverfettung oder kardiovaskuläre Risikofaktoren.

8. Verbindungen der allgemeinen Formel I nach Anspruch 3 zu deren Verwendung als Arzneimittel, das zur Behandlung und/oder Verhinderung von pathologischen Zuständen vorgesehen ist, die mit Lipidstörungen der Haut und mit entzündlichen oder mikrobiell bedingten Komplikationen verbunden sind.

9. Verbindungen der allgemeinen Formel I nach Anspruch 3 zu deren Verwendung als Arzneimittel, das zur Behandlung und/oder Verhinderung von Unregelmäßigkeiten der Produktion und/oder Sekretion von Sebum vorgesehen ist, die mit einem Hyperandrogenismus assoziiert sind.

10. Verbindungen der allgemeinen Formel I nach Anspruch 3 zu deren Verwendung als Arzneimittel, das zur Behandlung und/oder Verhinderung von Akne, Psoriasis, Hirsutismus, Rosazea, seborrhoischer Dermatitis, Hyperseborrhoe oder Ekzem vorgesehen ist.

11. Verbindungen der allgemeinen Formel I nach Anspruch 3 zu deren Verwendung als Arzneimittel, das zur Behandlung und/oder Verhinderung von Krebs vorgesehen ist.

12. Verbindungen der allgemeinen Formel I nach Anspruch 3 zu deren Verwendung als Arzneimittel, das zur Behandlung und/oder Verhinderung von flüssigen Tumoren und/oder soliden Tumoren vorgesehen ist, wie Melanome, kolorektale Karzinome, Lungen-, Prostata-, Blasen-, Brust-, Gebärmutter-, Speiseröhren-, Magen-, Bauchspeicheldrüsen- und Leberkrebserkrankungen, Eierstockkrebserkrankungen, Leukämien, insbesondere Lymphome und Myelome, HNO-Tumoren und Hirnkrebserkrankungen.

13. Pharmazeutische Zusammensetzung, **dadurch gekennzeichnet, dass** sie eine Verbindung der allgemeinen Formel I, wie in einem der Ansprüche 1 bis 2 definiert, als Wirkstoff enthält.

14. Pharmazeutische Zusammensetzung, **dadurch gekennzeichnet, dass** sie eine definierte Verbindung der allgemeinen Formel I, wie in Anspruch 13 definiert, in Verbindung mit einem beliebigen geeigneten Hilfsstoff enthält.

15. Pharmazeutische Zusammensetzung nach einem der Ansprüche 13 oder 14 in Verbindung mit einem Diabetesmittel, wie Biguanide, verschiedene Insulinformen, Sulfonylharnstoffe, Meglitinide, PPA-Modulatoren, alpha-Glucosidase-Hemer, DPP-4-Hemmer, Amylin-Analoga, Analoga von glucagonähnlichem Peptid-1, SGLT2-Hemmer oder 11β-HSD1-Hemmer.

16. Pharmazeutische Zusammensetzung nach einem der Ansprüche 13 oder 14 in Verbindung mit einem Mittel gegen Adipositas, wie Orlistat oder Sibutramin.

17. Pharmazeutische Zusammensetzung nach einem der Ansprüche 13 oder 14 in Verbindung mit einer Verbindung, die bei der Behandlung oder Verhinderung von pathologischen Zuständen, die mit Lipidstörungen der Haut und mit entzündlichen oder mikrobiell bedingten Komplikationen verbunden sind, oder Unregelmäßigkeiten der Produktion und/oder Sekretion von Sebum, die mit einem Hyperandrogenismus assoziiert sind, verwendet wird, wie Retinoide, Antibiotika, antibakteriell wirkende Substanzen oder antiandrogen wirkende Substanzen.

18. Pharmazeutische Zusammensetzung nach einem der Ansprüche 13 oder 14 in Verbindung mit anderen Arzneimitteln gegen Krebs, die zytotoxisch und/oder zytostatisch sind, wie Platinderivate, Taxane, Vinkas, 5-FU, um die therapeutische Wirksamkeit im Hinblick auf die Behandlung von refraktären Tumoren mit den gewöhnlichen Therapeutika zu verbessern.

19. Pharmazeutische Zusammensetzung nach einem der Ansprüche 13 oder 14, **dadurch gekennzeichnet, dass** sie in einer Form dargereicht wird, die zur topischen Verabreichung geeignet ist.

## Claims

1. Compounds having general formula I 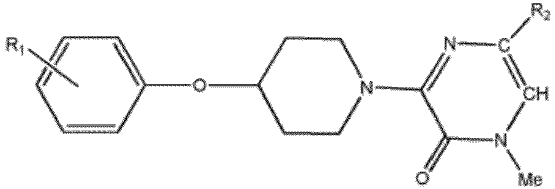 wherein
- R₁ represents one or a plurality of substituents chosen from trifluoromethyl, F, Cl, Br, methyl and nitro,
- R₂ represents Cl or H
as well as the pharmaceutically acceptable salts thereof.

2. Derivatives having general formula I according to claim 1, **characterised in that** they are selected from:
1. 5-Chloro-3-[4-(2-chloro-phenoxy)-piperidin-1-yl]-1-methyl-1H-pyrazin-2-one
2. 5-Chloro-1-methyl-3-[4-(2-trifluoromethyl-phenoxy)-piperidin-1-yl]-1H-pyrazin-2-one
3. 5-Chloro-3-[4-(2-chloro-5-trifluoromethyl-phenoxy)-piperidin-1-yl]-1-methyl-1H-pyrazin-2-one
4. 5-Chloro-1-methyl-3-(4-o-tolyloxy-piperidin-1-yl)-1H-pyrazin-2-one
5. 1-Methyl-3-(4-o-tolyloxy-piperidin-1-yl)-1H-pyrazin-2-one
6. 1-Methyl-3-[4-(2-trifluoromethyl-phenoxy)-piperidin-1-yl]-1H-pyrazin-2-one
7. 5-Chloro-3-[4-(2-chloro-5-fluoro-phenoxy)-piperidin-1-yl]-1-methyl-1H-pyrazin-2-one

3. Compounds having general formula I such as defined according to one of claims 1 and 2 for the use thereof as a medicinal product.

4. Compounds having general formula I as defined according to any of claims 1 and 2 for the use thereof as a cosmetic active ingredient.

5. Compounds having general formula I according to claim 3, for the use thereof as an SCD-1 enzyme inhibitor.

6. Compounds having general formula I according to claim 3, for the use thereof as a medicinal product for treating and/or preventing diseases requiring SCD-1 enzyme activity inhibitors.

7. Compounds having general formula I according to claim 3, for the use thereof as a medicinal product for treating and/or preventing diseases such as obesity, type 2 diabetes, diabetic dyslipidaemia, hypertriglyceridaemia, hypercholesterolaemia, metabolic syndrome, atherosclerosis, liver steatosis or cardiovascular risks.

8. Compounds having general formula I according to claim 3, for the use thereof as a medicinal product for treating and/or preventing pathological conditions associated with skin-related lipid disorders and inflammatory and bacterial complications.

9. Compounds having general formula I according to claim 3, for the use thereof as a medicinal product for treating and/or preventing sebum production and/or secretion disorders associated with hyperandrogenism.

10. Compounds having general formula I according to claim 3, for the use thereof as a medicinal product for treating and/or preventing acne, psoriasis, hirsutism, rosacea, seborrheic dermitis, hyperseborrhoea, or eczema.

11. Compounds having general formula I according to claim 3, for the use thereof as a medicinal product for treating and/or preventing cancer.

12. Compounds having general formula I according to claim 3, for the use thereof as a medicinal product for treating and/or preventing liquid tumours and/or solid tumours, such as melanomas, colorectal cancer, lung, prostate, bladder, breast, uterine, oesophageal, stomach, pancreatic, liver, ovarian cancers, leukaemia particularly lymphomas and myelomas, ENT-related cancer and brain cancer.

13. Pharmaceutical composition **characterised in that** it contains, as an active ingredient, a compound having general formula I as defined according to one of claims 1 to 2.

14. Pharmaceutical composition **characterised in that** it contains a defined compound having general formula I as defined according to claim 13 in association with any suitable excipient.

15. Pharmaceutical composition according to any of claims 13 or 14, in association with an anti-diabetic drug such as biguanides, various forms of insulin, sulphonylureas, meglitinides, PPAR modulators, les alpha-glucosidase inhibitors, DPP-4 inhibitors, amylin analogues, glucagon-like peptide-1 analogues, SGLT2 inhibitors or 11β-HSD1 inhibitors.

16. Pharmaceutical composition according to any of claims 13 or 14, in association with an anti-obesity drug such as orlistat or sibutramine.

17. Pharmaceutical composition according to any of claims 13 or 14, with a compound suitable for use for treating or preventing pathological conditions associated with skin-related lipid disorders and inflammatory and bacterial complications or sebum production and/or secretion disorders associated with hyperandrogenism such as retinoids, antibiotics, antibacterials, or antiandrogens.

18. Pharmaceutical composition 13 or 14, in association with other anticancer medication, whether they are cytotoxic and/or cytostatic, such as platinum derivatives, taxanes, vincas, 5-FU, to increase therapeutic efficacy for treating refractory tumours to routine treatments.

19. Pharmaceutical composition according to any of claims 13 or 14, **characterised in that** it is in a form suitable for topical administration.
